# EUROPÄISCHE PATENTSCHRIFT

(11) **EP 3 122 756 B1**
(45) Veröffentlichungstag und Bekanntmachung des Hinweises auf die Patenterteilung: **04.12.2019**
(21) Anmeldenummer: 15712797.8
(22) Anmeldetag: 26.02.2015
(51) Int. Cl.: C07F 7/00, C07F 9/58, C07F 9/6503, C07F 9/6506, C07F 9/6509, C07F 9/6533, C07F 9/655, C07F 9/6558, C07F 9/6561, C07F 9/6574, C07C 309/47, C07C 309/50, C07D 401/04, C07D 401/10, C07D 403/10, C07D 407/12, C07D 413/04, C07H 19/00, C07D 417/10, C07D 417/12

(54) **ANORGANISCH-ORGANISCHE HYBRIDVERBINDUNG**
INORGANIC-ORGANIC HYBRID COMPOUND
COMPOSÉ HYBRIDE INORGANIQUE-ORGANIQUE

(30) Priorität: 28.03.2014 DE 102014004512
(43) Veröffentlichungstag der Anmeldung: 01.02.2017
(73) Patentinhaber: Karlsruher Institut für Technologie, 76131 Karlsruhe (DE); Max-Planck-Gesellschaft zur Förderung der Wissenschaften e.V., 80539 München (DE); Georg-August-Universität Stiftung öffentlichen Rechts, Universitätsmedizin, 37075 Göttingen (DE)
(72) Erfinder: HECK, Joachim, 76547 Sinzheim (DE); POSS, Marieke, 76227 Karlsruhe (DE); REICHARDT, Holger, 37085 Göttingen (DE); NAPP, Joanna, 37124 Rosdorf (DE); ALVES, Frauke, 37075 Göttingen (DE); STÜHMER, Walter, 37077 Göttingen (DE); FELDMANN, Claus, 76275 Ettlingen (DE)
(74) Vertreter: Müller-Boré & Partner Patentanwälte PartG mbB
(86) Internationale Anmeldenummer: PCT/EP2015/000454
(87) Internationale Veröffentlichungsnummer: WO 2015/144282

(56) Entgegenhaltungen:
- EP-A1- 2 799 060
- WO-A1-2009/100800
- WO-A1-2011/044671
- WO-A2-01/89494
- JP-A- S5 967 294

## Beschreibung

Die vorliegende Erfindung betrifft eine anorganisch-organische Hybridverbindung als Ionenverbindung, bestehend aus einem anorganischen Kation und einem organischen Wirkstoffanion sowie gegebenenfalls einem organischen Fluoreszenzfarbstoffanion. Basierend auf einer einfachen, wasserbasierten Synthese ist eine breite Substanzpalette der erfindungsgemäßen anorganisch-organischen Hybridverbindung mit breiter Wirkstoff- und Therapiebasis verfügbar (z.B. entzündliche oder immunologische Erkrankungen wie Rheuma, Arthritis, Osteoporose, Multiple Sklerose; neurologische Erkrankungen wie Epilepsie oder Schizophrenie; Tumorerkrankungen; Infektionskrankheiten (bakteriell, viral, parasitär) wie Malaria, Tuberkulose oder Mykosen; Herz-Kreislauferkrankungen wie Angina Pectoris oder koronare Ablagerungen; zur Schmerztherapie). Zudem ist in einfacher Weise eine Kombination von Wirkstofffreisetzung mit optischer Detektion möglich. Die erfindungsgemäßen anorganisch-organischen Hybridverbindungen vereinen Therapie (Wirkstofffreisetzung) und Diagnostik (optische Detektion der Hybride sowie CT- oder MRI-basierte Detektion). Unter physiologischen Bedingungen wird der Wirkstoff sehr langsam über einen Zeitraum von einigen Stunden bis zu einigen Tagen freigesetzt und kann gezielt am Wirkort seine Wirkung entfalten, wobei die Hybridverbindung vollständig abgebaut wird.

Die gezielte Freisetzung von Pharmazeutika (Drug Delivery) mit Nanopartikeln stellt eine große Herausforderung für interdisziplinäre Wissenschaften dieser Zeit dar. Aktuell wird hier eine große Zahl an unterschiedlichen Material- und Therapiekonzepten für unterschiedlichste Krankheitsbilder verfolgt. Weit verbreitet ist die Einkapselung von Wirkstoffen in Vesikeln, meso- oder nanoskaligen Hohlkugeln oder Polymerkapseln. Darüber hinaus werden Wirkstoffe an Nanopartikel wie SiO₂, Au, Quantendots oder Polymernanopartikel gebunden. Alternativ können Wirkstoffe auch in Nanopartikel eingebettet werden, wobei SiO₂ und Polymere die gebräuchlichsten Matrixmaterialien sind. Nachteile der derzeit diskutierten Materialien und Lösungsansätze sind die zum Teil sehr geringe Menge an Wirkstoff in Bezug auf die Nanopartikel insgesamt (z.B. 1% Wirkstoff eingekapselt in 99% SiO₂ als Nanopartikelmatrix), die nicht vollständige Abbaubarkeit bzw. potentielle Toxizität der Nanopartikel nach Freisetzung des Wirkstoffs (beispielsweise bleiben SiO₂-Nanopartikel zurück). Darüber hinaus sind die Materialkonzepte und die Synthese der Materialien komplex und sehr aufwendig. Dies ist insbesondere für multifunktionelle Nanopartikel der Fall, die neben der Wirkstofffreisetzung auch eine analytische Erkennung ermöglichen.

US 2011/0064775 A1 beschreibt die Einkapselung von Nanopartikeln (z.B. Fe₃O₄ in Ausführungsbeispiel 1) oder eines organischen Fluoreszenzfarbstoffmoleküls (z.B. Fluorescein in Ausführungsbeispiel 2 bzw. 3) oder eines organischen Wirkstoffmoleküls (z.B. Doxorubicin in Ausführungsbeispiel 4) in einer metallorganischen Koordinationsverbindung, bestehend aus einem Zinksalz (z.B. Zinknitrat) und 1,4-Bis(Imidazol-1-ylmethyl)benzol (Bix) als Koordinations-Ligand. Die beschriebenen Verbindungen werden z.B. in absolutem (d.h. wasserfreiem) Ethanol hergestellt. WO 2009/100800 A1 beschreibt Anorganisch-organische Hybridverbindungen zur therapeutischen Verwendung.

US 8,779,175 B2 beschreibt wasserlösliche Koordinationskomplexe, die ein Wirkstoffmolekül beinhalten.

DE 20 2006 024 289 A1 offenbart Molekülanionen, die an Schichten von schichtbildenden Metallhydroxiden der Formel M²⁺₁₋ₓM³⁺ₓ(OH)₂(Zⁿ⁻)_{x/n} gebunden sind. Das strukturgebende Metallhydroxid wird aus den Kationen *M*^{2+/3+} und aus Hydroxidionen (OH) gebildet, die nicht fluoreszieren und keinen Wirkstoff darstellen.

Zusammenfassend können als Nachteile von Drug-Delivery-Systemen und Materialien gemäß dem Stand der Technik folgende Punkte angeführt werden:
- aufwendige chemische Synthese, Mehrstufenprozesse;
- komplexe Materialsysteme wie Kern-Schale-Strukturen und aufwendige Modifizierung der Partikeloberfläche;
- geringe Menge an Wirkstoff bezogen auf die Gesamtmasse der Nanopartikel;
- toxische bzw. unter physiologischen Bedingungen nicht vollständig oder nur sehr langsam abbaubare/ausscheidbare Bestandteile;
- Wirksamkeit eines komplexen Materialsystems nur gegen ein Krankheitsbild;
- Wirkstoff nur oberflächlich an ein Nanopartikel gebunden und daher in Suspension durch Partikelstöße leicht abreibbar;
- Auftreten von Nebenwirkungen;
- zu schnelle oder zu langsame Freisetzung der jeweiligen Pharmazeutika.

Vor diesem Hintergrund ist es die Aufgabe der vorliegenden Erfindung, ein einfaches Materialkonzept bei gleichzeitig sehr breitem Anwendungsspektrum bereitzustellen, welches über eine sehr einfache Synthese zugänglich ist, eine große Menge an Wirkstoff pro Nanopartikel ermöglicht und gleichzeitig eine optische Erkennung zulässt.

Diese Aufgabe wird durch die in den Ansprüchen gekennzeichneten Ausführungsformen gelöst.

Insbesondere wird eine anorganisch-organische Hybridverbindung als Ionenverbindung, aufgebaut aus einem anorganischen Kation und einem organischen Wirkstoffanion sowie gegebenenfalls einem organischen Fluoreszenzfarbstoffanion, bereitgestellt, wobei die Verbindung eine molare Löslichkeit von ≤ 10⁻² mol/l in Wasser aufweist und einen Partikeldurchmesser im Bereich von 1 bis 100 nm aufweist. Wirkstoffanion und Fluoreszenzfarbstoffanion, wenn vorhanden, enthalten jeweils mindestens eine Phosphat-, Phosphonat-, Sulfat-, Sulfonat-, Carbonat- oder Carboxylatgruppe als funktionelle Gruppe, die in Verbindung mit dem anorganischen Kation die Bildung einer in Wasser schwerlöslichen Verbindung und damit die Bildung von Nanopartikeln ermöglichen. Die erfindungsgemäße anorganisch-organische Hybridverbindung enthält vorzugsweise ein Wirkstoffanion sowie ein Fluoreszenzfarbstoffanion, so dass die anorganisch-organische Hybridverbindung einen Wirkstoff freisetzen kann und aufgrund der Fluoreszenz des Fluoreszenzfarbstoffanions in Zellen, Gewebe, Organen durch Lichtemission lokalisiert werden kann. Bevorzugt erfolgt die Anregung der Hybridverbindung mit sichtbarem Licht. Bevorzugt erfolgt die Emission der Hybridverbindung im sichtbaren bis infraroten Spektralbereich des Lichts.

Unter "Wirkstoffanion" wird ein Stoff, der als Mittel zur Heilung oder zur Verhütung menschlicher oder tierischer Krankheiten bezeichnet wird, sowie ein Stoff, der dazu bestimmt ist, im oder am menschlichen oder tierischen Körper zur Erstellung einer ärztlichen Diagnose oder zur Wiederherstellung, Besserung oder Beeinflussung der menschlichen oder tierischen Körperfunktionen angewandt zu werden, verstanden.

Das Wirkstoffanion kann dabei gegen sehr unterschiedliche Krankheitsbilder eingesetzt werden. Bevorzugt erfolgt der Einsatz des Wirkstoffanions gegen chronische Entzündungen/Asthma/Rheuma/Arthritis/Multiple Sklerose, Entzündungen allgemein, Tumorerkrankungen, Malaria, Tuberkulose, Angina Pectoris oder koronare Ablagerungen. Vorzugsweise kann das Wirkstoffanion unter physiologischen Bedingungen zeitverzögert über einen Bereich von einigen Stunden bis zu einigen Tagen freigesetzt werden. Bevorzugt erfolgt die Freisetzung einfach durch Hydrolyse unter physiologischen Bedingungen oder in Gegenwart von Phosphatasen durch Esterspaltung. Die erfindungsgemäße anorganisch-organische Hybridverbindung zeichnet sich auch dadurch aus, dass deren Bestandteile nicht allergen bzw. nicht toxisch sind und unter physiologischen Bedingungen vollständig abgebaut und/oder ausgeschieden werden.

Das in die erfindungsgemäße anorganisch-organische Hybridverbindung eingebaute, organische Wirkstoffanion unterliegt keiner wesentlichen Beschränkung, sofern es mindestens eine Phosphat-, Phosphonat-, Sulfat-, Sulfonat-, Carbonat- oder Carboxylatgruppe, vorzugsweise Phosphat-, Phosphonat-, Sulfat- oder Sulfonatgruppe, als funktionelle Gruppe, die in Verbindung mit dem anorganischen Kation die Bildung einer in Wasser schwerlöslichen Verbindung und damit die Bildung von Nanopartikeln ermöglichen, aufweist. Wie bereits vorstehend aufgeführt, sind solche Wirkstoffanionen bevorzugt, die gegen chronische Entzündungen/Rheuma/Arthritis/Multiple Sklerose, Entzündungen allgemein, Tumorerkrankungen, Malaria, Tuberkulose, Angina Pectoris oder koronare Ablagerungen wirken. Solche Wirkstoffe sind dem Fachmann bekannt. Im Rahmen der vorliegenden Erfindung können beispielhaft Acetaminophenphosphat, Betamethasonphosphat, Dexamethasonphosphat, Uridinmonophosphat, 5'-Fluor-2'-deoxyuridin-5'-monophosphat (FdUMP), Methylprednisolonphosphat, Triamcinolonphosphat, Estronphosphat, Testosteronphosphat, Estramustinphosphat, Codeinphosphat, Clindamycinphosphat, Thiaminpyrophosphat, Tiaminphosphat; Aracytidinmonophosphat, Cyclic-3',5'adenosinmonophosphat, Vidaribinphosphat, 9-[9-(Phosphonomethoxy)ethoxy]adenin, Fospropofol, Fosphenytoin, Phosphoryloxymethyloxymethylphenytoin, Phosphoryloxymethylphenylbutazon, Phosphoryloxymethyloxymethylphenylbutazon, Phosphoryloxymethylphenindion, Phosphoryloxymethyloxymethylphenindion, N-Phosphonooxymethylcinnarizin, N-Phosphonooxymethylloxapin, N-Phosphonooxymethylamiodaron, Alendronat, Canrenoat, Doxycyclin Hydrat, Doxorubicin Hydrochlorid, Aztreonam, Tigemonam, D-Glukosamin-6-sulfat, Colistin methansulfat, Cefsulodin, Fosamprenavir, Tenofovir, Adefovir, Combretastatin A-4 phosphat, Folsäure, Fosphenytoin, 2-Mercaptoethansulfonat / Mesna, Fosfomycin, Glyphosat, Glufosinat, Zolendronat, Aminotrimethylenphosphonsäure, Diethylentriaminpenta(methylenphosphonsäure), Ethylendiamintetra(methylenphosphonsäure), Fosbretabulin, α-Tocopherol phosphate, VAPOL hydrogenphosphate, Pyridoxal-5'-phosphate-6-(2'-naphthylazo-6'-nitro-4',8'-disulfonate), (11bR)-2,6-Di-9-phenanthrenyl-4-hydroxy-dinaphtho[2,1-d:1',2'-f] [1,3,2]dioxaphosphepin-4-oxide, 8-Bromo-cyclic adenosine diphosphate ribose, Phytinsäure, Glucose-6-phosphat bzw. andere Phosphorsäureester von Zuckern oder natürlich vorkommende und synthetische Nukleotide (z.B. Adenosinmonophosphat (AMP), Adenosindiphosphat (ADP), Adenosintriphosphat (ATP), Guanosinmonophosphat (GMP), Guanosindiphosphat (GDP), Guanosintriphosphat (GTP), Cytidinmonophosphat (CMP), Cytidindiphosphat (CDP), Cytidintriphosphat (CTP), Uridinmonophosphat (UMP), Uridindiphosphat (UDP), Uridintriphosphat (UTP), Desoxyadenosinmonophosphat (dAMP), Desoxyadenosindiphosphat (dADP), Desoxyadenosintriphosphat (dATP), Desoxyguanosinmonophosphat (dGMP), Desoxyguanosindiphosphat (dGDP), Desoxyguanosintriphosphat (dGTP), Desoxycytidinmonophosphat (dCMP), Desoxycytidindiphosphat (dCDP), Desoxycytidintriphosphat (dCTP), Desoxythymidinmonophosphat (dTMP), Desoxythymidindiphosphat (dTDP) oder Desoxythymidintriphosphat (dTTP)) eingesetzt werden. Darüber hinaus können auch organische Wirkstoffe eingesetzt werden, die als solche keine Phosphat-, Phosphonat-, Sulfat-, Sulfonat, Carbonat- oder Carboxylatgruppe aufweisen, indem diese mit mindestens einer von diesen funktionellen Gruppen modifiziert werden. Entsprechende Verfahren der Funktionalisierung solcher organischer Wirkstoffe sind dem Fachmann bekannt.

Im Rahmen der vorliegenden Erfindung ist das anorganische Kation aus Mg²⁺, Ca²⁺, Sr²⁺, Ba²⁺, Zn²⁺, Zr⁴⁺, [ZrO]²⁺, [HfO]²⁺, Sc³⁺, Y³⁺, Gd³⁺, La³⁺, Fe³⁺, Bi³⁺ oder einem Lanthanoid ausgewählt. Besonders bevorzugt sind die Kationen Mg²⁺, Ca²⁺, [ZrO]²⁺ oder La³⁺.

Im Rahmen der vorliegenden Erfindung ist es möglich, die Detektion der erfindungsgemäßen anorganisch-organischen Hybridverbindungen nicht nur optisch über die Fluoreszenz des Fluoreszenzfarbstoffanions, sondern in Gegenwart schwerer oder magnetischer anorganischer Kationen (z.B. Ba²⁺, [ZrO]²⁺, [HfO]²⁺, Gd³⁺, La³⁺, Fe³⁺, Bi³⁺) auch durch Röntgenabsorption oder magnetische Messungen durchzuführen.

Die erfindungsgemäße anorganisch-organische Hybridverbindung kann auch so verstanden werden, dass sie eine anorganische Matrix und eine organische Wirkstoffverbindung umfasst, wobei die anorganische Matrix aus einer anorganischen Verbindung, ausgewählt aus der Gruppe, bestehend aus Metallphosphaten, einschließlich den Hydrogenphosphaten und Dihydrogenphosphaten, Metalloxidphosphaten, Metallphosphonaten, Metallsulfaten, Metallsulfonaten, Metallcarbonaten oder Metallcarboxylaten, aufgebaut ist, wobei die anorganische Verbindung ein Kation, ausgewählt aus Mg²⁺, Ca²⁺, Sr²⁺, Ba²⁺, Zn²⁺, Zr⁴⁺, [ZrO]²⁺, [HfO]²⁺, Sc³⁺, y³⁺, Gd³⁺, La³⁺, Fe³⁺, Bi³⁺ oder einem Lanthanoid, enthält, wobei die organische Wirkstoffverbindung ein oder mehrere funktionelle Gruppen, ausgewählt aus Phosphat-, Phosphonat-, Sulfat-, Sulfonat, Carbonat- oder Carboxylatgruppen, aufweist, über welche die organische Wirkstoffverbindung in die anorganische Matrix eingebaut ist. Insofern die Wirkstoffverbindung und der optionale Fluoreszenzfarbstoff über deren funktionale Gruppe in die anorganische Matrix mittels ionischer Bindung eingebaut ist, ist demgemäß die funktionelle Gruppe, beispielhaft Phosphat, dann der anorganischen Matrix zuzurechnen, d.h. es liegt keine eigenständige Phosphatgruppe an der Wirkstoffverbindung vor.

Der Einbau sowohl der organischen Wirkstoffverbindung als auch des organischen Fluoreszenzfarbstoffs in die Hybridverbindung bzw. anorganische Matrix erfolgt über die anionischen funktionellen Gruppen, d.h. mittels ionischer Bindung. Die vorliegende Erfindung umfasst solche anorganisch-organische Hybridverbindungen, welche molare Mengen an Wirkstoffverbindung enthalten, als auch "verdünnte" Varianten. Beide Varianten lassen sich durch einfaches Mischen wässriger Lösungen der Ausgangsmaterialien erhalten.

Gemäß der vorliegenden Erfindung kann die anorganisch-organische Hybridverbindung das Wirkstoffanion, gegebenenfalls zusammen mit dem Fluoreszenzfarbstoffanion, in molaren Mengen enthalten, wobei die molare Menge an Wirkstoffanion und Fluoreszenzfarbstoffanion unter Berücksichtigung der jeweiligen Ionenladungen in stöchiometrischem Verhältnis zum anorganischen Kation steht. In der anorganisch-organischen Hybridverbindung kann das Verhältnis von Wirkstoffanion zu Fluoreszenzfarbstoffanion aber auch variiert werden.

In einer weiteren Ausführungsform der vorliegenden Erfindung ist die anorganisch-organische Hybridverbindung weiter mit einem Antikörper oder Peptid wie z.B. Antikörper und Antikörperfragmente, Nanobodies, Diabodies, Peptid-Aptamere oder mit einem Oligonukleotid wie z.B. Aptamere oder ähnlichen Molekülen funktionalisiert, um die anorganisch-organischen Hybridnanopartikel und den enthaltenen Wirkstoff *in vivo* an einen spezifischen Wirkort zu schleusen und hier anzureichern. Aufgrund der wasserbasierten Synthese der anorganisch-organischen Hybridverbindungen ist diese Kopplung mit Antikörpern oder ähnlichen Molekülen besonders einfach und schonend.

In einer bevorzugten Ausführungsform der vorliegenden Erfindung enthält die anorganisch-organische Hybridverbindung, wie vorstehend bereits ausgeführt, weiterhin einen organischen Fluoreszenzfarbstoff, d.h. ein entsprechendes Anion davon, welcher eine oder mehrere funktionelle Gruppen, ausgewählt aus Phosphat-, Phosphonat-, Sulfat-, Sulfonat, Carbonat- oder Carboxylatgruppen, aufweist, über welche der Fluoreszenzfarbstoff(anion) in die lonenverbindung bzw. anorganische Matrix eingebaut ist. Vorzugsweise ist der organische Fluoreszenzfarbstoff aus der Gruppe, bestehend aus 1,1'-Diethyl-2,2'-cyaniniodid, 1,2-Diphenylacetylen, 1,4-Diphenylbutadien, 1,4-Diphenylbutadien, 1,6-Diphenylhexatrien, 2,5-Diphenyloxazol, 2-Methylbenzoxazol, 4',6-Diamidino-2-phenylindol (DAPI), 4-(Dicyanomethylen)-2-methyl-6-(p-dimethylaminostyryl)-4H-pyran (DCM), 4-Dimethylamino-4'-nitrostilben, 5,10,15-Triphenylcorrol, 5,10,15-Tris(pentafluorophenyl)corrol, 5,10-Diarylchlorin, 5,10-Diarylkupferchlorin, 5,10-Diarylkupferoxochlorin, 5,10-Diarylmagnesiumoxochlorin, 5,10-Diaryloxochlorin, 5,10-Diarylzinkchlorin, 5,10-Diarylzinkooxochlorin, 7-Benzylamino-4-nitrobenz-2-oxa-1,3-diazol, 7-Methoxycumarin-4-essigsäure, 9,10-Bis(phenylethinyl)anthracen, 9,10-Diphenylanthracen, Acridinorange, Acridingelb, Adenin, Anthracen, Anthrachinon, Auramin O, Azobenzol, Bakteriochlorophyll A, Benzochinon, Betacarotin, Bilirubin, Biliverdindimethylester, Biphenyl, Bis(5-mesityldipyrrinato)zink, Bis(5-phenyldipyrrinato)zink, Borsubphtalocyaninchlorid, Chlorin E6, Chlorophyll A, Chlorophyll B, cis-Stilben, Cumarin sowie dessen Derivaten, Cresylviolettperchlorat, Cryptocyanin, Kristallviolett, Cytosin, Dansylglycin, Diprotonated-tetraphenylporphyrin, Eosin sowie dessen Derivaten, Ethyl-(p-dimethylamino)-benzoat, Ferrocen, Fluorescein sowie dessen Derivaten, beispielsweise Methylfluorescein, Resorufin, Amaranth, Aluminium(III)-Phthalocyanin-Chlorid Tetrasulfonsäure, Trypan Blau, Guanin, Hematin, Histidin, Hoechst 33258, Indocarbocyanine sowie dessen Derivaten, Lucifergelb CH, Magnesiumoctaethylporphyrin, Magnesiumphthalocyanin, Magnesiumtetramesitylporphyrin, Magnesiumtetraphenylporphyrin, Malachitgrün, Merocyanin, N,N'-Difluoroboryl-1,9-dimethyl-5-(4-iodophenyl)-dipyrrin, N,N'-Difluoroboryl-1,9-dimethyl-5-[(4-(2-trimethylsilylethinyl), N,N'-Difluoroboryl-1,9-dimethyl-5-phenydipyrrin, Tetraphenylporphyrin, Naphthalin, Nilblau, Nilrot, Octaethylporphyrin, Oxacarbocyanin sowie dessen Derivaten, Oxazin sowie dessen Derivaten, p-Quarterphenyl, p-Terphenyl, Perylen sowie dessen Derivaten, Phenol, Phenylalanin, Phenyldipyrrin, Pheophorbid, Phthalocyanin, Pinacyanoliodid, Piroxicam, Porphin, Proflavin, Protoporphyrin-IX-dimethylester, Pyren, Pyropheophorbid sowie dessen Derivaten, Pyrrol, Chinin, Rhodamin sowie dessen Derivaten, Riboflavin, Bengalrot, Squarylium dye III, TBP-beta-octa(COOBu)-Fb, TBP-beta-octa(COOBu)-Pd, TBP-beta-octa(COOBu)-Zn, TBP-mesotetraphenyl-beta-octa(COOMe)-Fb, TBP-meso-tetraphenyl-beta-octa(COOMe)-Pd, TBP-meso-tetraphenyl-beta-octa(COOMe)-Zn, TCPH-meso-tetra(4-COOMephenyl)-Fb, TCPH-meso-tetra(4-COOMe-phenyl)-Pd, TCPH-meso-tetra(4-COOMe-phenyl)-Zn, Tetra-t-butylazaporphin, Tetra-t-butylnaphthalocyanin, Tetrakis(2,6-dichlorophenyl)porphyrin, Tetrakis(o-aminophenyl)porphyrin, Tetramesitylporphyrin, Tetraphenylporphyrin, Tetraphenylsapphyrin, Thiacarbocyanin sowie dessen Derivaten, Thymin, trans-Stilben, Tris(2,2'-bipyridyl)ruthenium(II), Tryptophan, Thyrosin, Uracil, Vitamin B12, Zinkoctaethylporphyrin, Phthalocyanin sowie dessen Derivaten, Porphyrin sowie dessen Derivaten, z.B. Tetra(o-amidophosphonatophenyl)porphyrin, und Umbelliferon, ausgewählt. Dabei sind die organischen Fluoreszenzfarbstoffe, die als solche keine Phosphat-, Phosphonat-, Sulfat-, Sulfonat, Carbonat- oder Carboxylatgruppe aufweisen, mit mindestens einer von diesen funktionellen Gruppen modifiziert (z.B: Phenylumbelliferonphosphat (PUP), Methylfluoresceinphosphat (MFP), Resorufinphosphat (RRP), Dyomics-647-uridinphosphat (DUT)). Entsprechende Verfahren zum Funktionalisieren von solchen organischen Fluoreszenzfarbstoffen sind dem Fachmann bekannt.

In einer weiteren bevorzugten Ausführungsform der vorliegenden Erfindung ist der organische Fluoreszenzfarbstoff aus der Gruppe, bestehend aus Riboflavin-5'-monophosphatnatriumsalz, Fluorescein, Resorufin, Amaranth, Rhodamin, Perylen, Cumarin und Umbelliferon, letztere mit mindestens einer Phosphat-, Phosphonat-, Sulfat-, Sulfonat, Carbonat- oder Carboxylatgruppe funktionalisiert, ausgewählt. Beispielhaft kann hier Phenylumbelliferonphosphat angeführt werden.

Die anorganisch-organische Hybridverbindung ist schwerlöslich. Im Rahmen der vorliegenden Erfindung werden unter schwerlöslichen Verbindungen solche Verbindungen verstanden, die eine molare Löslichkeit von ≤ 10⁻² mol/l aufweisen. Vorzugsweise weisen die schwerlöslichen Verbindungen eine molare Löslichkeit von ≤ 10⁻⁴ mol/l auf. Dies ist hinsichtlich der Synthese der erfindungsgemäßen anorganisch-organischen Hybridverbindungen vorteilhaft, da sich so die anorganisch-organische Hybridverbindung, welche die Wirkstoffverbindung sowie den gegebenenfalls vorgesehenen Fluoreszenzfarbstoff enthält, aus löslichen Vorläuferverbindungen ausfällen lässt.

Üblicherweise weist die anorganisch-organische Hybridverbindung eine röntgenamorphe Struktur auf. Dies ist hinsichtlich einer vereinfachten Synthese vorteilhaft, da amorphe Nanopartikel ohne größeren synthetischen Aufwand erhalten werden können.

In einer bevorzugten Ausführungsform der vorliegenden Erfindung ist die anorganisch-organische Hybridverbindung weiter mit einem oder mehreren Kationen und/oder Anionen dotiert. Durch eine Dotierung ist es möglich, die Lumineszenzeigenschaften der erfindungsgemäßen Hybridverbindung zu modifizieren, da nach Anregung des organischen Fluoreszenzfarbstoffes ein vollständiger oder partieller Energieübertrag auf die Dotierung erfolgt, so dass nachfolgend eine von der Dotierung stammende Emission zu beobachten ist. Es ist weiterhin möglich, dass die Dotierung eine veränderte Anregung der erfindungsgemäßen Hybridverbindung bewirkt. Die Dotierung kann in jedem geeigneten Konzentrationsbereich erfolgen. Vorzugsweise liegt die Dotierung in einem Konzentrationsbereich von 5 ppm bis 50 mol-% vor, besonders bevorzugt in einem Konzentrationsbereich von 0,1 bis 5,0 mol-%. Die anorganisch-organische Hybridverbindung ist vorzugsweise mit einem Lanthanoid, ausgewählt aus Ce, Pr, Nd, Sm, Eu, Gd, Tb, Dy, Ho, Er, Tm, Yb oder Lu, einem Übergangsmetall, ausgewählt aus Cr, Mn, Cu, Zn, Y, Ag oder Cd, einem Hauptgruppenelement, ausgewählt aus Sn, Sb, Pb oder Bi, oder einem komplexen Anion, ausgewählt aus [VO₄]³⁻, [MoO₄]³⁻ oder [WO₄]³⁻, dotiert.

Die erfindungsgemäße Hybridverbindung kann jede geeignete Partikelgröße aufweisen. In einer bevorzugten Ausführungsform ist die erfindungsgemäße Hybridverbindung nanoskalig und weist einen Partikeldurchmesser im Bereich von 1 bis 100 nm auf. Besonders bevorzugt ist ein Partikeldurchmesser im Bereich von 1 bis 20 nm. Darüber hinaus weist die erfindungsgemäße Hybridverbindung vorzugsweise eine nahezu monodisperse Größenverteilung im Bereich von ±30%, besonders bevorzugt im Bereich von ±5%, auf. Weiterhin weist die erfindungsgemäße Hybridverbindung vorzugsweise einen geringen Agglomerationsgrad auf, besonders bevorzugt mit einer Größenverteilung im Bereich von ±30%, noch mehr bevorzugt im Bereich von ±5%, auf. Im Stand der Technik sind geeignete Verfahren zur Bestimmung des Partikeldurchmessers und der monodispersen Größenverteilung bekannt. Gemäß der vorliegenden Erfindung können jeweils unterschiedliche Anregungs- und Emissionseigenschaften bei gleicher Hybridverbindung durch die Wahl und den Einbau eines entsprechenden organischen Fluoreszenzfarbstoffes eingestellt werden. Vorzugsweise liegt die Anregung der erfindungsgemäßen Hybridverbindung im Bereich von 100 bis 800 nm und die Emission im Bereich von 200 bis 2000 nm. In der Regel erfolgt eine Anregung durch eine Leuchtdiode oder einen Laser, welche sichtbares bis nah-infrarotes Licht emittieren (d.h. 450 bis 800 nm) und eine Emission des organischen Fluoreszenzfarbstoffes bzw. der erfindungsgemäßen Hybridverbindung im sichtbaren Spektralbereich zwischen blau und infrarot (d.h. 450 bis 1400 nm). In einer anderen Ausführungsform erfolgt die Anregung in Form von UV-Licht (d.h. 100 bis 450 nm). Vorzugsweise nimmt bei der erfindungsgemäßen Hybridverbindung die Lumineszenzintensität unter Anregungsbedingungen über die Dauer der Anregung im Vergleich zu dem ungebundenen organischen Fluoreszenzfarbstoff weniger stark ab, besonders bevorzugt nimmt die Lumineszenzintensität über die Dauer der Anregung nicht ab, insbesondere bei Anregung mit einer Leuchtdiode. So nimmt bei Einwirkung mit UV-Licht die Lumineszenzintensität vorzugsweise um nicht mehr als 10% ab, bei Einwirkung mit Tageslicht um nicht mehr als 1%.

Die vorliegende Erfindung betrifft ferner ein Verfahren zur Herstellung der erfindungsgemäßen Hybridverbindung, umfassend die Schritte
(a) das Bereitstellen einer Lösung einer organischen Wirkstoffverbindung, die eine oder mehrere funktionelle Gruppen, ausgewählt aus Phosphat-, Phosphonat-, Sulfat-, Sulfonat, Carbonat- oder Carboxylatgruppen, aufweist, wobei die Lösung gegebenenfalls weiter mindestens ein Anion, ausgewählt aus Phosphat-, Phosphonat-, Sulfat-, Sulfonat, Carbonat- oder Carboxylat, enthält,
(b) das Bereitstellen einer Lösung eines löslichen Metallsalzes, enthaltend Metallkationen, ausgewählt aus Mg²⁺, Ca²⁺, Sr²⁺, Ba²⁺, Zn²⁺, Zr⁴⁺, [ZrO]²⁺, [HfO]²⁺, Sc³⁺, y³⁺, Gd³⁺, La³⁺, Fe³⁺, Bi³⁺ oder einem Lanthanoid,
(c) das Vereinigen der beiden Lösungen unter Rühren, um die Hybridverbindung auszufällen, und
(d) das Isolieren und/oder Aufreinigen der ausgefällten Hybridverbindung.

Der Schritt (a) des erfindungsgemäßen Verfahrens umfasst das Bereitstellen einer Lösung der organischen Wirkstoffverbindung. Ferner kann diese Lösung gegebenenfalls weiter mindestens ein Anion, ausgewählt aus Phosphat, Phosphonat, Sulfat, Sulfonat, Carbonat oder Carboxylat, enthalten. Wenn eingesetzt, so kann dieses Anion zusammen mit einem Kation in der Form eines gelösten Salzes vorliegen, beispielsweise als gelöstes Alkalimetallsulfat, Alkalimetallphosphat, Alkalimetallcarboxylat, Alkalimetallcarbonat. Vorzugsweise handelt es sich bei dem Alkalimetall um Natrium oder Kalium. Das Anion kann auch in Form der korrespondierenden Säure in der Lösung vorliegen. In einer Ausführungsform der vorliegenden Erfindung enthält die Lösung zur Bereitstellung eines der vorstehend genannten Anionen eine Säure, ausgewählt aus der Gruppe, bestehend aus Schwefelsäure, Phosphorsäure oder einer Carbonsäure. Bei der Carbonsäure handelt es sich vorzugsweise um Ameisensäure, Essigsäure, Propionsäure oder Oxalsäure. Bei dem Carboxylat handelt es sich dementsprechend vorzugsweise um Formiat, Acetat oder Propionat. Besonders bevorzugt enthält die Lösung Phosphat als Anion und vorzugsweise wird Phosphorsäure zur Bereitstellung dieses Anions verwendet.

In einer bevorzugten Ausführungsform der vorliegenden Erfindung wird zu der in Schritt (a) bereitgestellten Lösung weiter ein organischer Fluoreszenzfarbstoff, welcher eine oder mehrere funktionelle Gruppen, ausgewählt Phosphat-, Phosphonat-, Sulfat-, Sulfonat, Carbonat- oder Carboxylatgruppe, aufweist, zugegeben.

Als Lösungsmittel kann jedes geeignete Lösungsmittel verwendet werden. Vorzugsweise wird als Lösungsmittel Wasser, isotones Wasser, ein physiologischer Puffer, ein Alkohol oder ein Gemisch aus mehreren dieser Lösungsmittel verwendet. Bevorzugte Alkohole zur Verwendung als Lösungsmittel sind Methanol, Ethanol, Propanol und Isopropanol.

Der Schritt (b) des erfindungsgemäßen Verfahrens umfasst das Bereitstellen einer Lösung eines löslichen Metallsalzes, welches Metallkationen enthält, die gleich oder verschieden sein können und aus Mg²⁺, Ca²⁺, Sr²⁺, Ba²⁺, Zn²⁺, Zr⁴⁺, [ZrO]²⁺, [HfO]²⁺, Sc³⁺, y³⁺, Gd³⁺, La³⁺, Fe³⁺, Bi³⁺ oder einem Lanthanoid ausgewählt sind. Als Lösungsmittel kann jedes geeignete Lösungsmittel verwendet werden. Vorzugsweise werden ebenfalls die vorstehend genannten Lösungsmittel, nämlich Wasser, isotones Wasser, ein physiologischer Puffer, Alkohole sowie Gemische aus mehreren dieser Lösungsmittel verwendet. In einer besonders bevorzugten Ausführungsform der vorliegenden Erfindung wird isotones Wasser oder ein physiologischer Puffer als Lösungsmittel verwendet. Als Metallsalz kann jedes Salz verwendet werden, das in dem verwendeten Lösungsmittel löslich ist. Geeignete Metallsalze sind dem Fachmann bekannt. Vorzugsweise können als Metallsalze die Halogenide, Nitrate und Sulfate der vorstehend genannten Metalle verwendet werden, sofern diese im jeweils verwendeten Lösungsmittel löslich sind. In einer besonders bevorzugten Ausführungsform der vorliegenden Erfindung wird Magnesiumdichlorid, Calciumdichlorid, Lanthantrichlorid oder Zirkonylchlorid als Metallsalz verwendet.

Der Schritt (c) des erfindungsgemäßen Verfahrens umfasst das Vereinigen der beiden Lösungen unter Rühren. Auf diese Weise wird die erfindungsgemäße Hybridverbindung ausgefällt. Bei dem Schritt des Vereinigens können die beiden Lösungen jede geeignete Temperatur aufweisen. In einer bevorzugen Ausführungsform der vorliegenden Erfindung weist zumindest eine der beiden Lösungen oder weisen beide Lösungen eine Temperatur im Bereich von Raumtemperatur bis 85°C auf, besonders bevorzugt eine Temperatur im Bereich von 40°C bis 75°C. Das Vereinigen der beiden Lösungen erfolgt vorzugsweise rasch, d.h. innerhalb eines Zeitraums von nicht mehr als 10 Sekunden, vorzugsweise innerhalb eines Zeitraums von nicht mehr als 5 Sekunden.

Der Schritt (d) des erfindungsgemäßen Verfahrens umfasst das Isolieren und/oder Aufreinigen der ausgefällten Hybridverbindung. Dieses Isolieren und/oder Aufreinigen kann durch alle geeigneten Verfahren erfolgen. Derartige Verfahren sind im Stand der Technik bekannt.

Vorzugsweise erfolgt das Isolieren und/oder Aufreinigen der Hybridverbindungspartikel durch ein Verfahren, ausgewählt aus der Gruppe, bestehend aus Zentrifugationstechniken, Dialysetechniken, Phasentransfertechniken, Chromatographietechniken, Ultrafiltrationstechniken, Waschtechniken und Kombinationen davon. Die vorstehend genannten Verfahren zur Isolierung und/oder Aufreinigung der Hybridverbindungspartikel können auch kombiniert und/oder mehrfach ausgeführt werden.

Die Figuren zeigen:

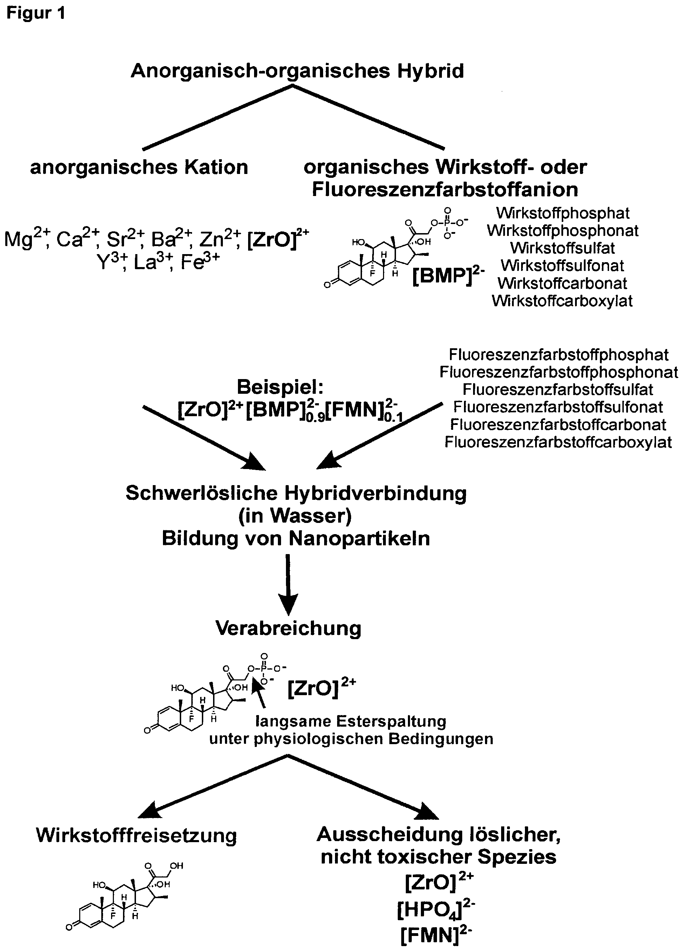

Figur 1 eine schematische Darstellung der Synthese und Verwendung einer anorganisch-organischen Hybridverbindung mit einem Wirkstoffanion und einem Fluoreszenzfarbstoffanion am Beispiel von [ZrO]²⁺[BMP]²⁻_{0.9}[FMN]²⁻_{0.1} (BMP: Betamethasonphosphat; FMN: Flavinmononukleotid);

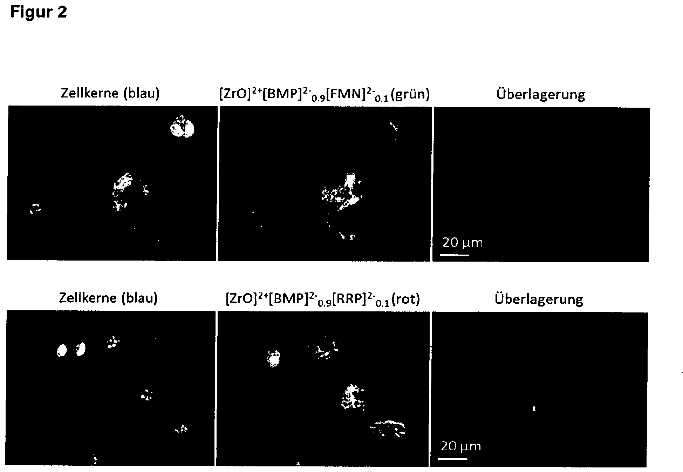

Figur 2 lichtmikroskopische Aufnahmen von anorganisch-organischen Hybridverbindungen mit einem Wirkstoffanion und einem Fluoreszenzfarbstoffanion in Makrophagen anhand der Fluoreszenz des Fluoreszenzfarbstoffanions am Beispiel von [ZrO]²⁺[BMP]²⁻_{0.9}[FMN]²⁻_{0.1} (grün) und von [ZrO]²⁺[BMP]²⁻_{0.9}[RRP]²⁻_{0.1} (rot) (BMP: Betamethasonphosphat; FMN: Flavinmononukleotid; RRP: Resorufinphosphat). Zellkerne sind angefärbt mit DAPI (blau);

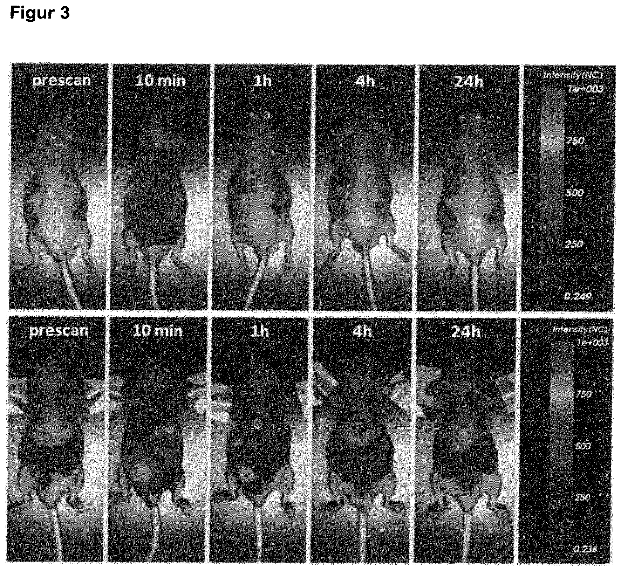

Figur 3 einen Nachweis der Verteilung von anorganisch-organischen Hybridverbindungen mit einem Wirkstoffanion und einem Fluoreszenzfarbstoffanion im Mausmodell nach intravenöser Verabreichung anhand der Fluoreszenz des Fluoreszenzfarbstoffanions am Beispiel von [ZrO]²⁺[BMP]²⁻_{0.95}[IRF]²⁻_{0.05} (BMP: Betamethasonphosphat; IRF: IR-emittierender Fluoreszenzfarbstoff);

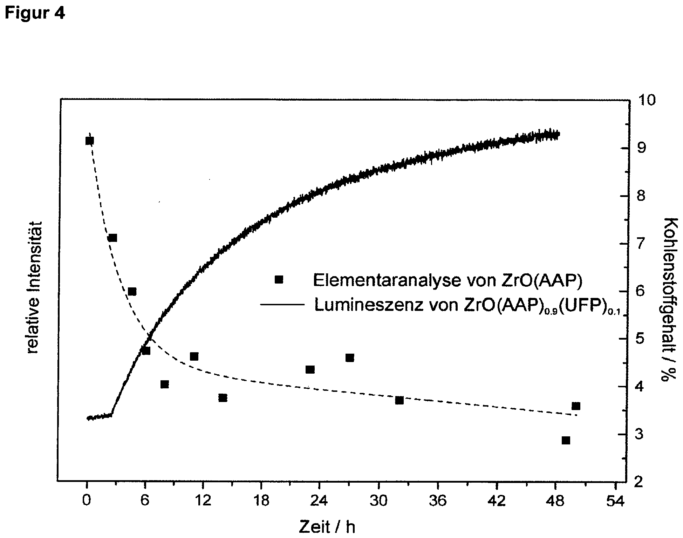

Figur 4 die Wirkstofffreisetzung aus anorganisch-organischen Hybridverbindungen mit einem Wirkstoffanion und einem Fluoreszenzfarbstoffanion am Beispiel von [ZrO]²⁺[AAP]²⁻_{0.9}[UFP]²⁻_{0.1}, wobei die Freisetzung durch Detektion der Fluoreszenz des freigesetzten Fluoreszenzfarbstoffs (UFP) sowie die Abnahme des Kohlenstoffgehalts in den verbleibenden Nanopartikeln gemäß Elementaranalyse gezeigt ist (AAP: Acetaminophenphosphat; UFP: Umbelliferonphosphat); und

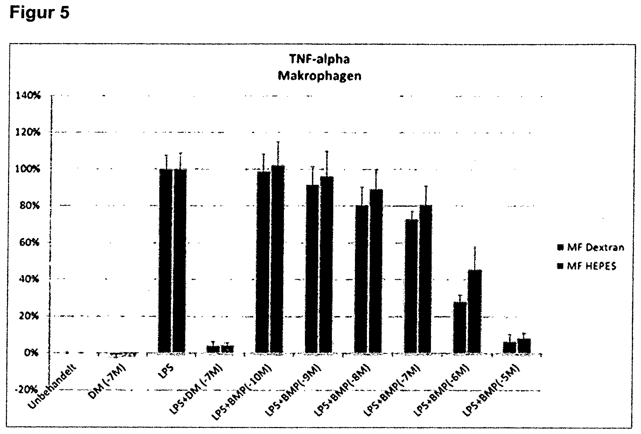

Figur 5 die Wirkstofffreisetzung aus anorganisch-organischen Hybridverbindungen mit einem Wirkstoffanion und einem Fluoreszenzfarbstoffanion am Beispiel von [ZrO]²⁺[BMP]²⁻_{0.9}[FMN]²⁻_{0.1}, wobei die Wirksamkeit von BMP als Entzündungshemmer in Lipopolysaccharid (LPS) stimulierten und aus Mäusen frisch isolierten Peritonealmakrophagen (MF) bei steigender Konzentration von 10⁻¹⁰ molar bis zu 10⁻⁵ molar im Vergleich zu Dexamethason (DM) gezeigt ist (BMP: Betamethasonphosphat; FMN: Flavinmononukleotid).

Unter physiologischen Bedingungen wird aus dem Wirkstoffanion der Wirkstoff sehr langsam über einen Zeitraum von einigen Stunden bis zu einigen Tagen freigesetzt und kann am Wirkort seine Wirkung entfalten. Dies ist in Figur 4 für die langsame Freisetzung in einer Laborsuspension gezeigt. Die Wirkstoffanionen können dabei aus einem sehr breiten Bereich bezüglich der Zusammensetzung gewählt werden und decken einen weiten Bereich an möglichen Krankheitsbildern ab. Neben der Freisetzung eines Wirkstoffs können die erfindungsgemäßen anorganisch-organischen Hybridverbindungen durch die Fluoreszenz eines zusätzlich eingebauten Fluoreszenzfarbstoffanions lokalisiert werden. Figur 3 zeigt, dass die Verteilung und Lokalisation der anorganisch-organischen Hybridverbindungen nach intravenöser Verabreichung *in vivo* im Mausmodell mittels nichtinvasiver Nahinfrarot-Bildgebung dargestellt werden kann. Figur 2 zeigt die Aufnahme und Anreicherung der anorganisch-organischen Hybridverbindungen in Zellen (Makrophagen). Figur 5 zeigt die Wirksamkeit von Betamethasonphosphat, das als Wirkstoff aus den anorganisch-organischen Hybridverbindungen freigesetzt wird und gemäß *in vitro* Assays in LPSstimulierten Makrophagen entzündungshemmend wirkt. Um die Nanopartikel *in vivo* an einen spezifischen Wirkort zu bringen, können die anorganisch-organischen Hybridnanopartikel zusätzlich mit einem gegen ein krankheitsspezifisches Target gerichtetes Peptid, Antikörper oder Oligonukleotid funktionalisiert werden.

Zusammenfassend weist die vorliegende Erfindung folgende Vorteile gegenüber dem Stand der Technik auf:
- Anorganisch-organische Hybridverbindungen als neuartiges Materialkonzept zur Wirkstoffverabreichung;
- vorzugsweise enthält die anorganisch-organische Hybridverbindung sowohl einen Wirkstoff für die Therapie als auch einen Fluoreszenzfarbstoff für die Diagnostik;
- die chemische Zusammensetzung ist in einem weiten Bereich wählbar, und zwar mit anorganischen Metallkationen, ausgewählt aus Mg²⁺, Ca²⁺, Sr²⁺, Ba²⁺, Zn²⁺, Zr⁴⁺, [ZrO]²⁺, [HfO]²⁺, Sc³⁺, Y³⁺, Gd³⁺, La³⁺, Fe³⁺, Bi³⁺ oder einem Lanthanoid, und einem organischen Anion, wobei es sich um einen Wirkstoff und gegebenenfalls zusätzlich um einen Fluoreszenzfarbstoff handelt, der jeweils eine Phosphat-, Phosphonat-, Sulfat-, Sulfonat, Carbonat- oder Carboxylatgruppe als funktionelle Gruppe trägt (daher als Wirkstoffanion bzw. Fluoreszenzfarbstoffanion bezeichnet);
- die anorganisch-organische Hybridverbindung ist durch einfache optische Detektion über die Fluoreszenz des Fluoreszenzfarbstoffanions in Zellen, Gewebe oder Organen möglich. Zusätzlich ist eine Detektion durch Röntgenabsorption oder magnetische Messungen in Gegenwart schwerer oder magnetischer anorganischer Kationen (z.B. Ba²⁺, [ZrO]²⁺, [HfO]²⁺, Gd³⁺, La³⁺, Fe³⁺, Bi³⁺) möglich;
- Wirkstoffanion und Fluoreszenzfarbstoffanion sind in der anorganisch-organischen Hybridverbindung in molaren Mengen enthalten, wobei die molare Menge an Wirkstoffanion und Fluoreszenzfarbstoffanion unter Berücksichtigung der jeweiligen Ionenladungen in stöchiometrischem Verhältnis zum anorganischen Kation steht. Zusätzlich kann das Verhältnis von Wirkstoffanion zu Fluoreszenzfarbstoffanion variiert werden;
- die anorganisch-organische Hybridverbindung ist in der Regel nicht kristallin (d.h. röntgenamorph). Die aufwendige Synthese kristalliner Materialien bzw. die Ausbildung von Kern-Schale-Strukturen ist nicht erforderlich. Dies vereinfacht die Synthese sehr stark. Zudem kann die Synthese durch einfache Fällung in wässriger Phase erfolgen, da die Hybridverbindungen in Wasser schwerlöslich sind. So lässt sich auch die Keimbildung und das Keimwachstum in Wasser gut kontrollieren, so dass Partikeldurchmesser unter 100 nm sehr leicht zugänglich sind;
- die anorganisch-organische Hybridverbindung kann sehr unterschiedliche Wirkstoffanionen enthalten und erlaubt daher einen Einsatz für ein sehr breites medizinisches Therapiegebiet (z.B. chronische Entzündungen/Rheuma/Arthritis/Multiple Sklerose, Entzündungen allgemein, Tumorerkrankungen, Malaria, Tuberkulose, Angina Pectoris oder koronare Ablagerungen). Der Wirkstoff wird aus der anorganisch-organischen Hybridverbindung unter physiologischen Bedingungen zeitverzögert über einen Bereich von einigen Stunden bis zu einigen Tagen freigesetzt. Auf diesem Wege kann eine bestimmte Wirkstoffdosis über einen längeren Zeitraum und unmittelbar am Wirkort freigesetzt werden. Nebenwirkungen bzw. der ungewünschte physiologische Abbau von Wirkstoff, zum Beispiel im Blut, kann herabgesetzt bzw. vermieden werden;
- ebenso kann die anorganisch-organische Hybridverbindung sehr unterschiedliche Fluoreszenzfarbstoffanionen enthalten. Typischerweise erfolgt die Anregung im Spektralbereich des sichtbaren Lichtes mit geeigneten Lasern oder Leuchtdioden (LEDs) unter Emission im Bereich des sichtbaren Lichtes bzw. im Infraroten;
- die anorganisch-organischen Hybridverbindungen zeichnen sich neben dem Wirkstoff durch nicht-allergene bzw. nicht-toxische Bestandteile aus, die unter physiologischen Bedingungen vollständig abgebaut ausgeschieden werden;
- um die anorganisch-organischen Hybridnanopartikel *in vivo* an einen spezifischen Wirkort zu schleusen und hier anzureichern, können diese zusätzlich mit einem gegen ein krankheitsspezifisches Target gerichteten Antikörper oder Peptid funktionalisiert sein. Aufgrund der wasserbasierten Synthese der anorganisch-organischen Hybridverbindungen ist auch diese Kopplung mit Antikörpern oder ähnlichen Molekülen besonders einfach und schonend.

Die in der vorstehend angeführten US 2011/0064775 offenbarten Verbindungen unterscheiden sich an zwei entscheidenden Stellen von den erfindungsgemäßen anorganisch-organischen Hybridverbindungen:
(i) Die erfindungsgemäßen anorganisch-organischen Hybridverbindungen sind aus einem Kation und zumindest einem Wirkstoffanion aufgebaut. Es liegt eine homogene Zusammensetzung vor. Die in US 2011/0064775 A1 beschriebenen Verbindungen sind heterogen, d.h. sie enthalten zwei klar voneinander trennbare Substanzen (vgl. Fig. 2 von US 2011/0064775 A1). US 2011/0064775 A1 beschreibt den Einschluss von Nanopartikeln bzw. von Molekülen in einem metallorganischen Koordinationspolymer (Zn-Bix). Dieses Koordinationspolymer (Zn-Bix) ist weder fluoreszenzaktiv noch ist es ein Wirkstoff. Die Matrix (Zn-Bix) ist im Hinblick auf die Funktion der Partikel (Fluoreszenz, Wirkstoff) somit funktionslos. Die Funktion wird im Gegensatz zu den erfindungsgemäßen anorganisch-organischen Hybridverbindungen von den eingeschlossenen Molekülen oder Nanopartikeln getragen. Damit ist die Gesamtmenge an aktiven Molekülen für das Gesamtsystem begrenzt.
(ii) Die in US 2011/0064775 A1 beschriebenen Verbindungen sind in Wasser löslich und müssen daher in absolutem, wasserfreiem Ethanol hergestellt werden. Demgegenüber sind die erfindungsgemäßen Hybridverbindungen in Wasser schwerlöslich. Dies ist essentiell, um die erfindungsgemäßen Hybridverbindungen in Wasser herstellen zu können bzw. diese in wässrigen Systemen (z.B. Medizin, d.h. in Zellflüssigkeit oder Blut) verwenden zu können.

Ebenso unterscheiden sich die in der vorstehend angeführten US 8,779,175 B2 offenbarten Verbindungen grundlegend von den erfindungsgemäßen anorganisch-organischen Hybridverbindungen:
(i) Die in US 8,779,175 B2 beschriebenen Verbindungen sind in Wasser löslich und müssen daher z.B. in absolutem, wasserfreien Methanol hergestellt werden (z.B. Magnesiumtopiramat, Spalte 21, Zeile 41ff.). Wird eine Synthese in Wasser durchgeführt, können allenfalls überschüssige Ausgangsmaterialien aufgrund ihrer geringen Löslichkeit in Wasser abgetrennt werden. Der Koordinationskomplex als Zielverbindung ist in Wasser hingegen löslich und kann als Feststoff nur durch Abdestillieren des Wassers (am Rotationsverdampfer) gewonnen werden (z.B. Calciumtopiramat). Demgegenüber sind die erfindungsgemäßen Hybridverbindungen in Wasser schwerlöslich. Dies ist essentiell, um die erfindungsgemäßen Hybridverbindungen in Wasser herstellen zu können bzw. diese in wässrigen Systemen (z.B. Medizin, d.h. in Zellflüssigkeit oder Blut) verwenden zu können.
(ii) Die in US 8,779,175 B2 erhaltenen Substanzen sind unstrukturierte Feststoffe, keine Nanomaterialien. Dies schränkt eine Anwendung in Dünnschichten (keine optische Transparenz) und in der Medizin (keine intravenöse Verabreichung) deutlich ein.

Im Gegensatz zu den in DE 20 2006 024 289 offenbarten Metallhydroxid-Verbindungen können die erfindungsgemäßen anorganisch-organischen Hybridverbindungen das Wirkstoffmolekül als einziges und ausschließliches Anion enthalten. Dies ist bei schichtbildenden Metallhydroxiden chemisch nicht möglich. Das Gerüst (schichtbildendes Hydroxid) ist im Hinblick auf die Funktion der Substanz (Fluoreszenz, Wirkstoff) funktionslos. Die Funktion wird im Gegensatz zu den erfindungsgemäßen anorganisch-organischen Hybridverbindungen von den angebundenen Molekülen oder Nanopartikeln getragen. Damit ist die Gesamtmenge an aktiven Molekülen für das Gesamtsystem begrenzt.

Gegenüber den vorstehenden Stand der Technik Dokumente kann in den erfindungsgemäßen anorganisch-organischen Hybridverbindungen die Menge an Fluoreszenz- bzw. Wirkstoffanion äquimolar zum jeweiligen Kation sein, was ein Vorteil ist, da damit extrem hohe Wirkstoff- bzw. Fluoreszenzfarbstoffgehalte erreicht werden können ([ZrO][BMP] enthält z.B. 81 Gew.-% des aktiven Wirkstoffs BMP). Ähnliches gilt z.B. für [ZrO]²⁺[FdUMP]²⁻. Die erfindungsgemäßen anorganisch-organischen Hybridverbindungen können in Wasser hergestellt werden und sind in Wasser schwerlöslich. Sind die Verbindungen nicht in Wasser schwerlöslich, können in Gegenwart von Wasser keine Nanopartikel hergestellt oder gelagert werden (wg. Auflösung). Eine Anwendung in Zellen bzw. in Blut ist nicht möglich, wenn die Verbindungen unter wässrigen Bedingungen leichtlöslich sind.

Die vorliegende Erfindung wird durch die nachstehenden, nicht-beschränkenden Beispiele weiter erläutert.

### Beispiele:

### Ausführungsbeispiel 1: [ZrO][BMP]_{0.9}[FMN]_{0.1}:

Die anorganisch-organische Hybridverbindung ZrO(BMP)_{0.9}(FMN)_{0.1} wird durch Mischen zweier Lösungen dargestellt. Lösung 1 enthält ZrOCl₂·8H₂O (5 mg) in demineralisiertem Wasser (2,5 ml). Lösung 2 enthält Natriumriboflavin-5'-monophosphat Dihydrat (2,4 mg) und Natriumbetamethasonphosphat (21,6 mg) in demineralisiertem Wasser (25 ml). Lösung 2 wurde auf 50 °C erwärmt und stark gerührt (ca. 1000 RPM). Anschließend wurde Lösung 1 schnell mit einer Spritze unter intensivem Rühren injiziert. Nach zweiminütigem Rühren wird der gelb gefärbte Feststoff durch Zentrifugieren (15 min bei 22500 U min⁻¹) abgetrennt. Die Nanopartikel werden zweimal in demineralisiertem Wasser (25 ml) resuspendiert und erneut zentrifugiert, um alle verbliebenen Salze zu entfernen. Schließlich werden stabile Suspensionen durch Resuspendieren der Nanopartikel in HEPES-Puffer (12 ml, 30 mmol/l, pH = 7,4) erhalten. Alternativ wird das Zentrifugat in demineralisiertem Wasser (3,1 ml) resuspendiert. Anschließend wird zu dieser Suspension unter Rühren eine Lösung aus Dextran 40 (3 ml, 1,6 mg/ml H₂O) getropft. Das verwendete demineralisierte Wasser wird in allen Fällen zuvor mit Hilfe eines sterilen Spritzenfilters (PA, 0,20 µm) vor Verwendung staub- und keimfrei gemacht. Man erhält die anorganisch-organische Hybridverbindung [ZrO][BMP]_{0.9}[FMN]_{0.1}, welches [ZrO]²⁺ als anorganisches Kation, das Wirkstoffanion [BMP]²⁻ sowie das Fluoreszenzfarbstoffanion [FMN]²⁻ enthält, als amorphe Nanopartikel mit einem Durchmesser von etwa 60 nm.

### Weitere Ausführungsbeispiele:

Analog zu Ausführungsbeispiel 1 können anstelle von Betamethasonphosphat [BMP]²⁻ als Wirkstoffanion andere Wirkstoffanionen sowie anstelle von Flavinmononukleotid [FMN]²⁻ als Fluoreszenzfarbstoffanion andere Fluoreszenzfarbstoffanionen in den erfindungsgemäßen anorganisch-organischen Hybridverbindungen verwendet werden, wie nachstehend beispielhaft aufgeführt; vgl. Verbindungen Nrn. 1 bis 45. Zusätzlich können andere Kationen als [ZrO]²⁺ wie Mg²⁺, Ca²⁺, Sr²⁺, Ba²⁺, Zn²⁺, [ZrO]²⁺, [HfO]²⁺, Sc³⁺, Y³⁺, Gd³⁺, La³⁺, Fe³⁺ oder Bi³⁺ gewählt werden, wobei aus anorganischem Kation und organischem Anion eine in Wasser schwerlösliche Hybridverbindung synthetisiert wird.

Angegeben sind nachfolgend Salze oder die freie Säure des jeweiligen funktionellen Anions, die alternativ beide verwendet werden können. Die angegebene Form entspricht einer üblichen, im Handel erhältlichen Form des betreffenden funktionellen organischen Anions, so wie es als Ausgangssubstanz für die Synthese erfindungsgemäßer anorganisch-organischer Hybridnanopartikel eingesetzt werden kann.

### 1. Acetaminophenphosphat

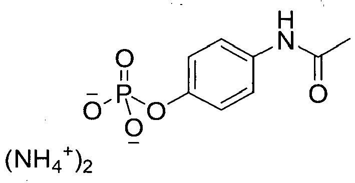

### 2. Betamethasonphosphat

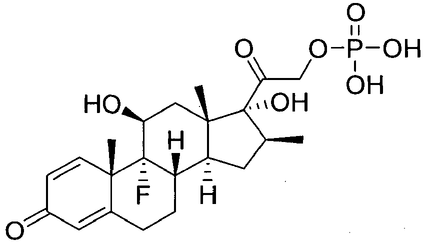

### 3. Dexamethasonphosphat

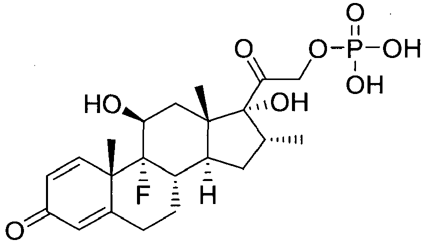

### 4. Methylprednisolonphosphat

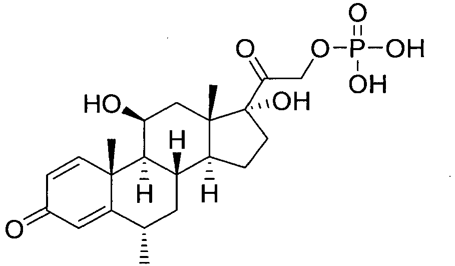

### 5. Triamcinolonphosphat

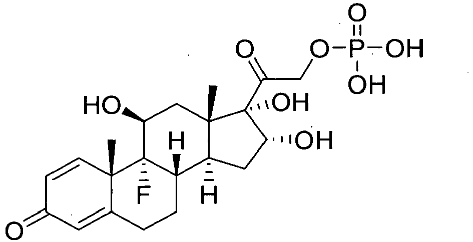

### 6. Estronphosphat

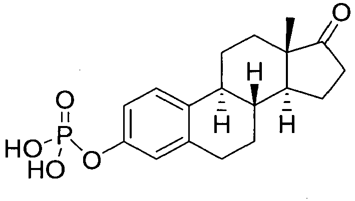

### 7. Testosteronphosphat

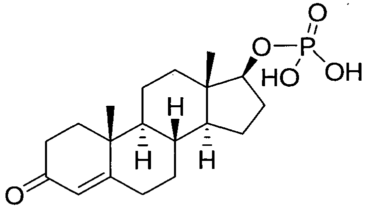

### 8. Estramustinphosphat

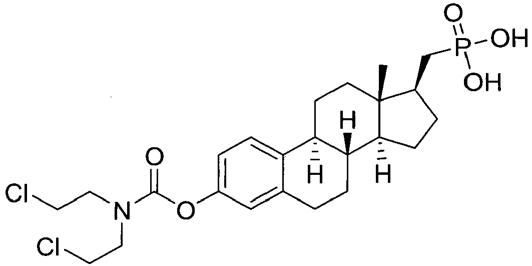

### 9. Codeinphosphat

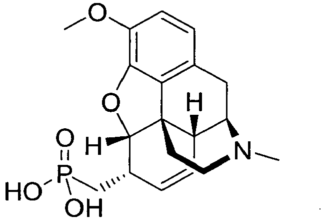

### 10. Clindamycin phosphat

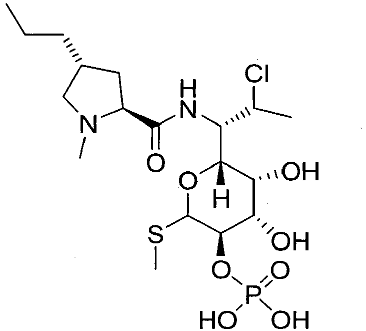

### 11. Thiaminpyrophosphat

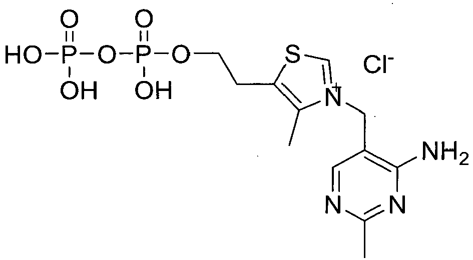

### 12. Thiaminphosphat

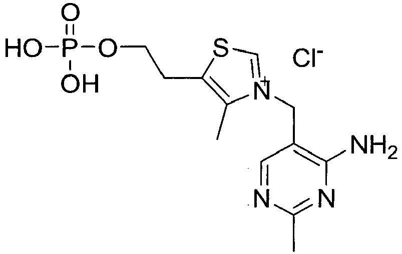

### 13. Aracytidinmonophosphat (ara-CMP)

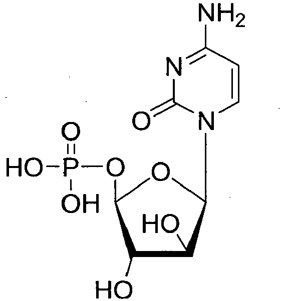

### 14. Cyclic-3',5'-adenosinmonophosphat

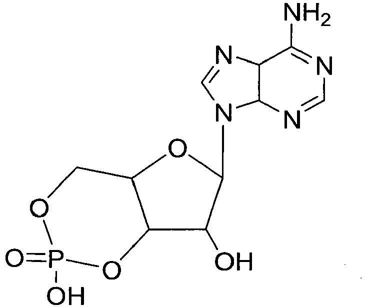

### 15. Vidaribinphosphat

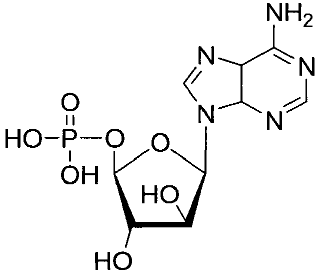

### 16. 9-[9-(Phosphonomethoxy)ethoxy]adenin

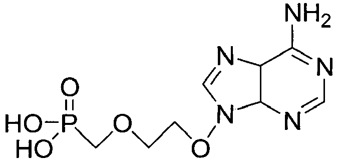

### 17. Fospropofol (Lusedra®)

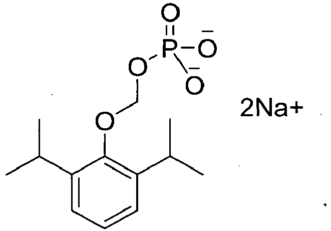

### 18. Fosphenytoin

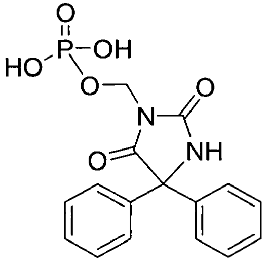

### 19. Phosphoryloxymethyloxymethylphenytoin

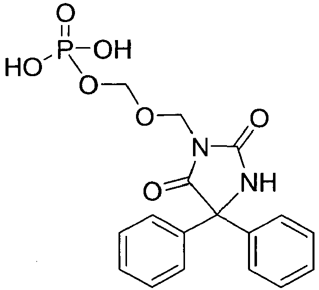

### 20. Phosphoryloxymethylphenylbutazon

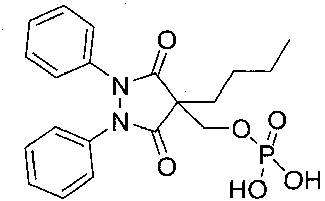

### 21. Phosphoryloxymethyloxymethylphenylbutazon

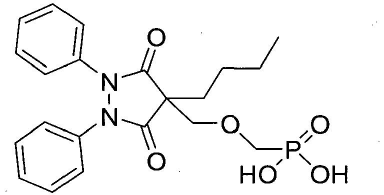

### 22. Phosphoryloxymethylphenindion

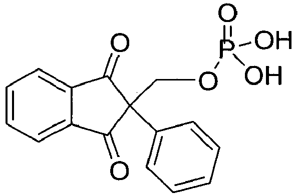

### 23. Phosphoryloxymethyloxymethylphenindion

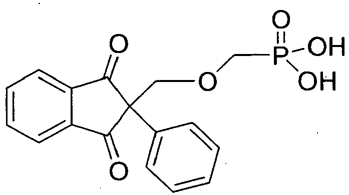

### 24. N-Phosphonooxymethylcinnarizin

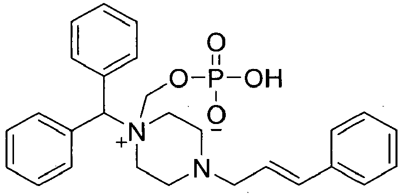

### 25. N-Phosphonooxymethylloxapin

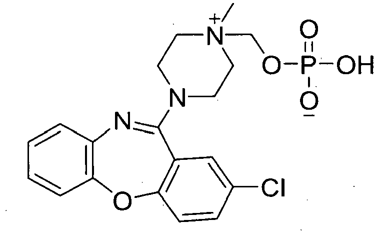

### 26. N-Phosphonooxymethylamiodaron

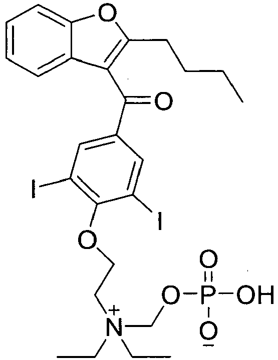

### 27. Alendronat

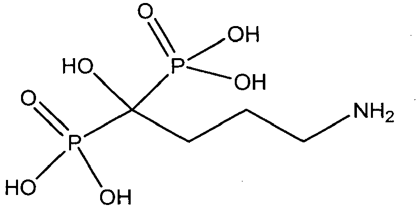

### 28. Resorufinphosphat

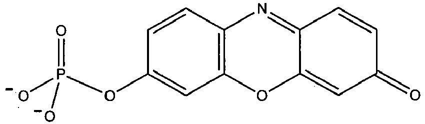

### 29. Brilliant Schwarz

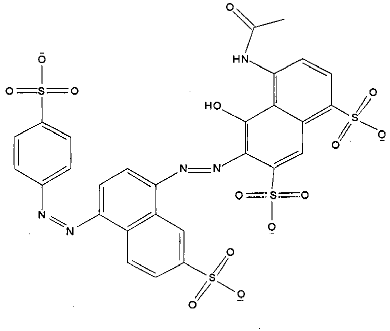

### 30. 3-Phenylumbelliferonphosphat

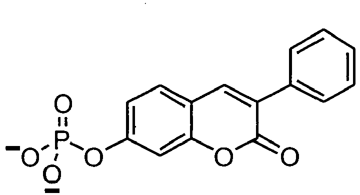

### 31. 3-O-Methylfluoresceinphosphat

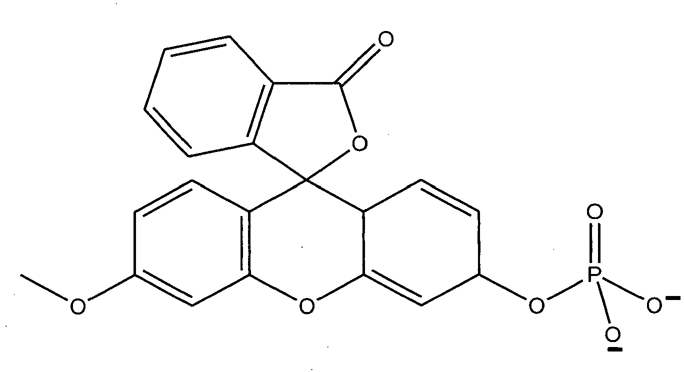

### 32. Amaranth / Acid Red 27 / Azorubin S

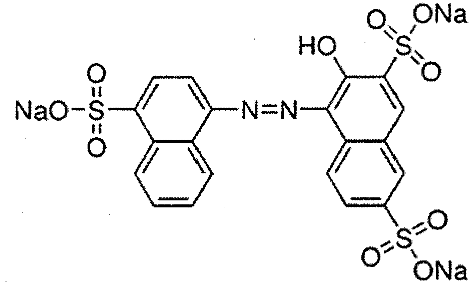

### 33. Cibacron Brilliant Red 3B-A / Reactive Red 4

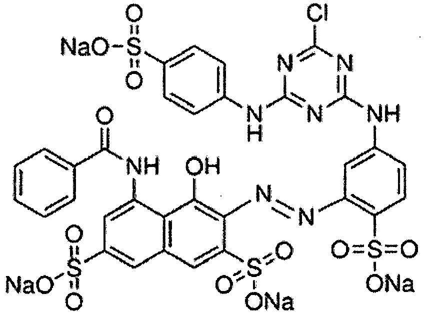

### 34. Acid Anthracene Red G

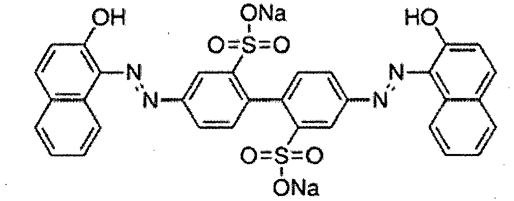

### 35. Nuclear Fast Red / Calcium Red / Kerntechrot

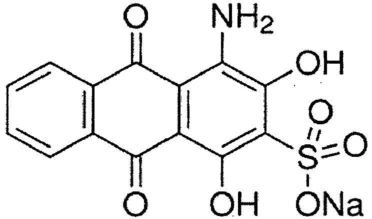

### 36. Kaliumcanrenoat

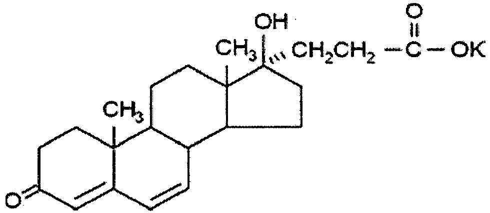

### 37. Doxycyclin Hydrat / Doxycyclin Hydrochlorid Hemiethanolat Hemihydrat

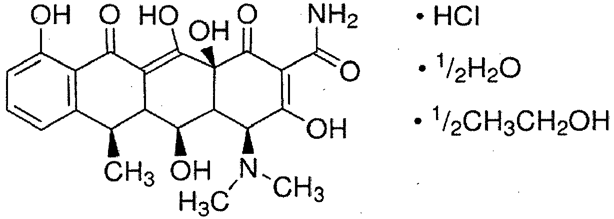

### 38. Calcein / Fluorexon / Fluorescein-bis(methyliminodiacetic acid)

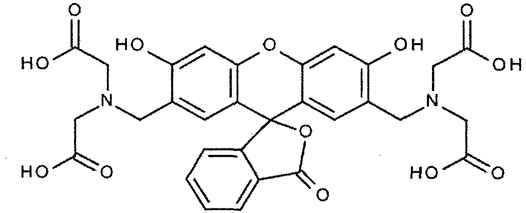

### 39. Nitrazingelb

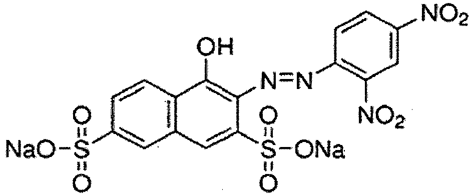

### 40. ABTS / 2,2'-Azino-bis(3-ethylbenzothiazoline-6-sulfonic acid) diammonium salt

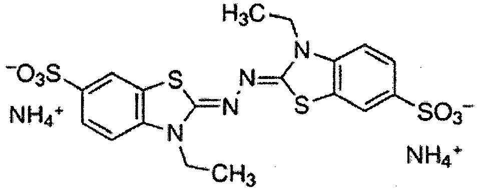

### 41. Doxorubicin Hydrochlorid

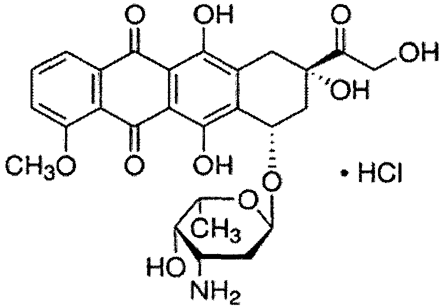

### 42. ANSA magnesium salt / 8-Anilino-1-naphthalenesulfonic acid hemimagnesium salt hydrate

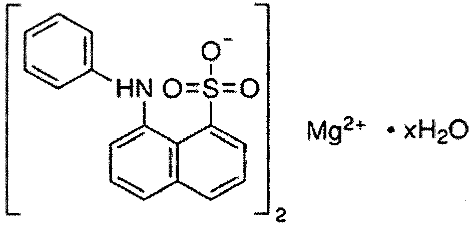

### 43. Indocyaningrün /Cardiogreen

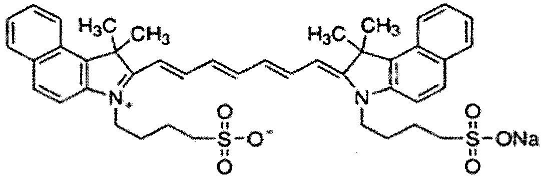

### 44. Lucifer Yellow CH Dilithium Salz

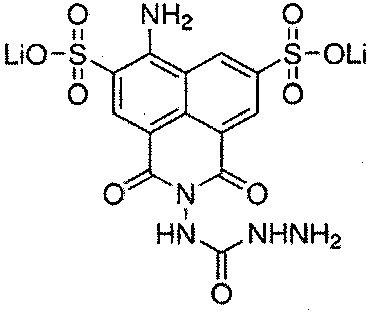

### 45. Fluorescent red 633

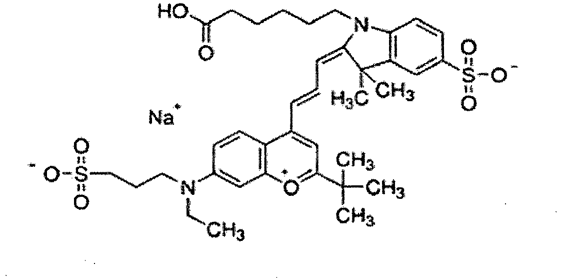

### 46. Aluminium(III)-Phthalocyanin-Chlorid Tetrasulfonsäure

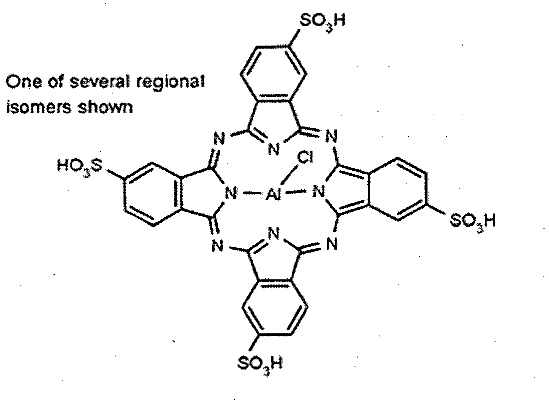

### 47. Aztreonam

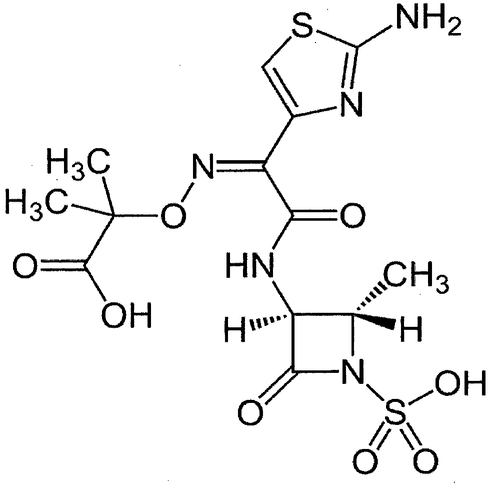

### 48. Tigemonam

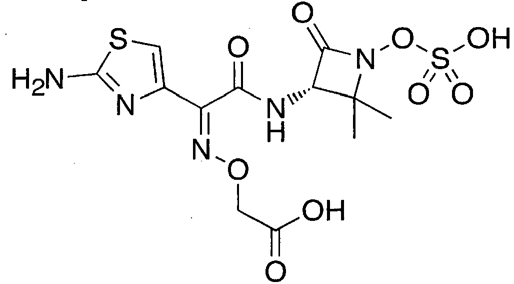

### 49. D-Glukosamin-6-sulfat

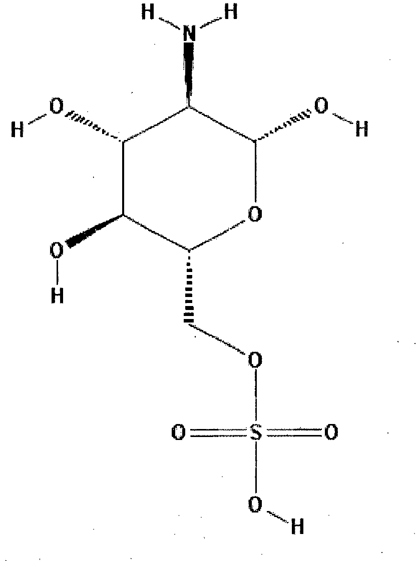

### 50. Colistin methansulfat

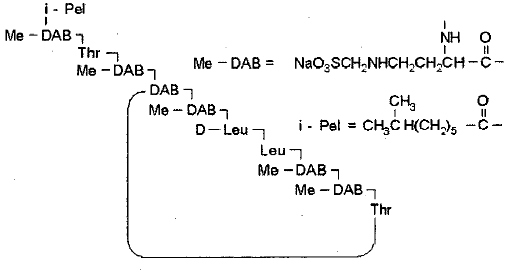

### 51. Cefsulodin

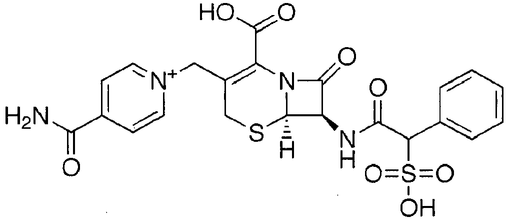

### 52. Fosamprenavir

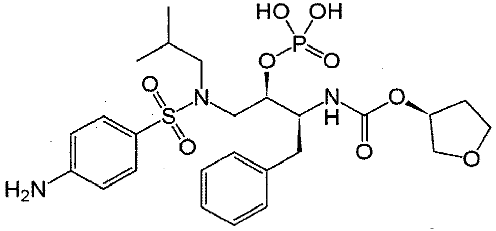

### 53. Tenofovir

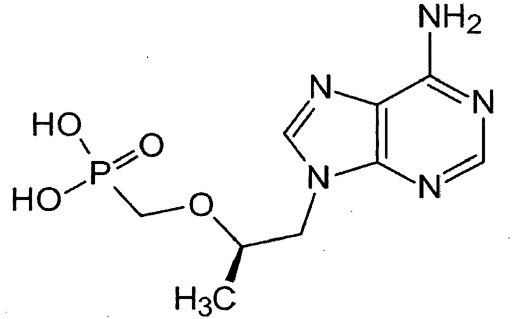

### 54. Adefovir

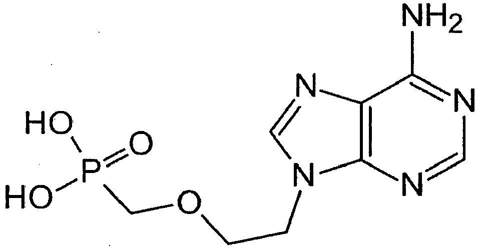

### 55. Combretastatin A-4 phosphat

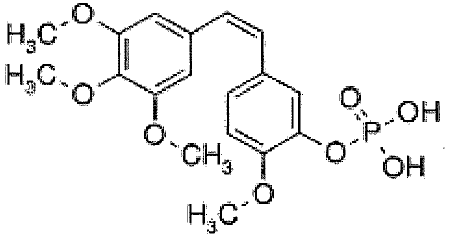

### 56. Folsäure

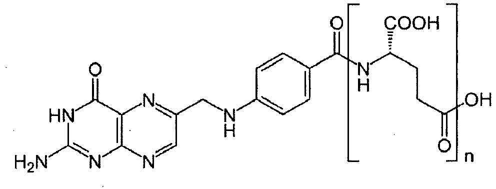

### 57. Fosphenytoin

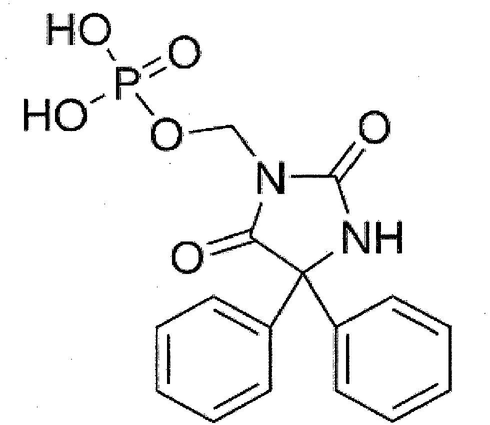

### 58. 2-Mercaptoethansulfonat / Mesna

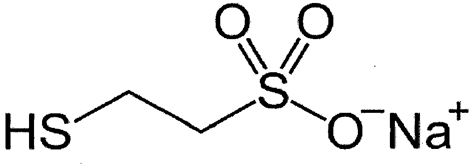

### 59. Fosfomycin

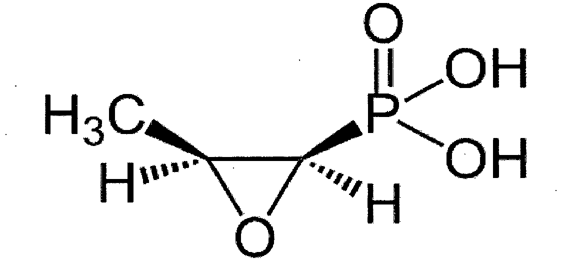

### 60. Glyphosat

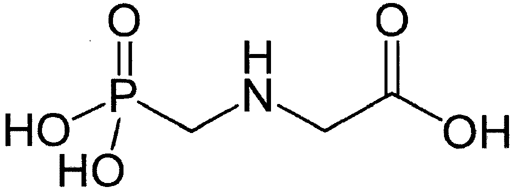

### 61. Glufosinat

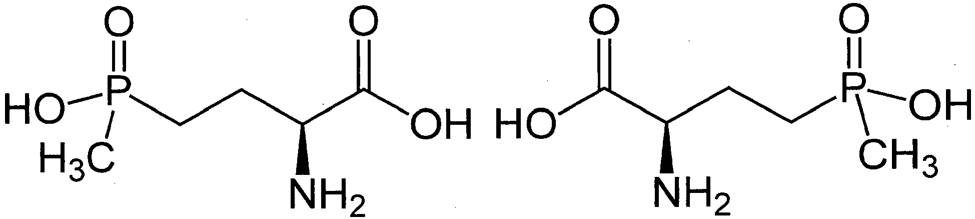

### 62. Zolendronat

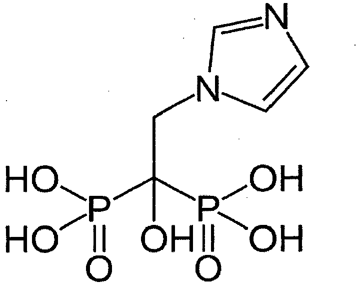

### 63. Aminotrimethylenphosphonsäure

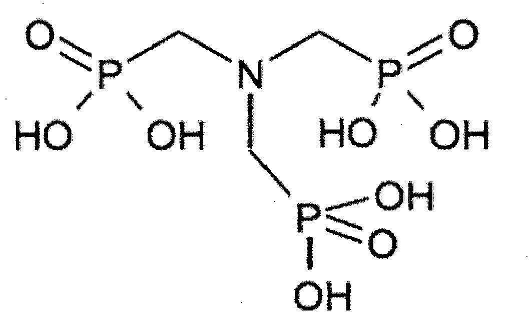

### 64. Diethylentriaminpenta(methylenphosphonsäure)

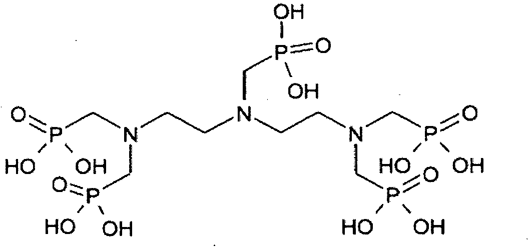

### 65. Ethylendiamintetra(methylenphosphonsäure)

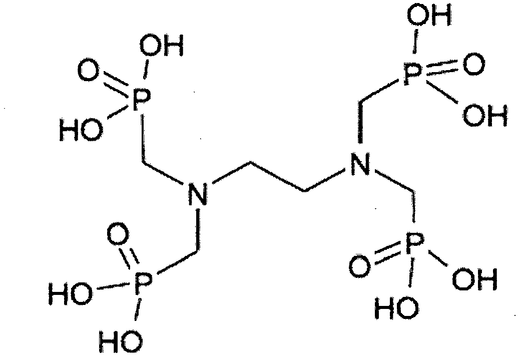

### 66. Fosbretabulin

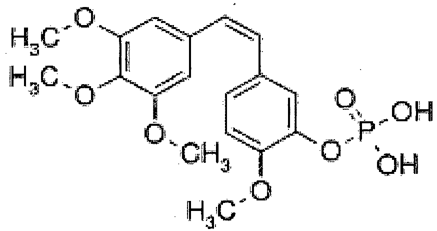

### 67. α-Tocopherol phosphat

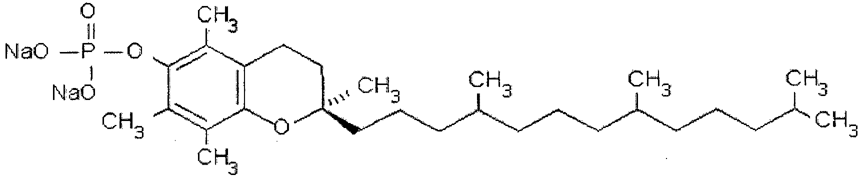

### 68. VAPOL hydrogenphosphat

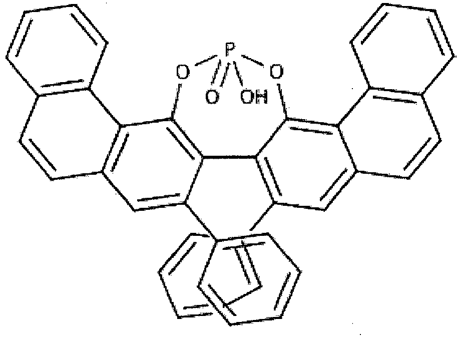

### 69. Pyridoxal-5'-phosphat-6-(2'-naphthylazo-6'-nitro-4',8'-disulfonat)

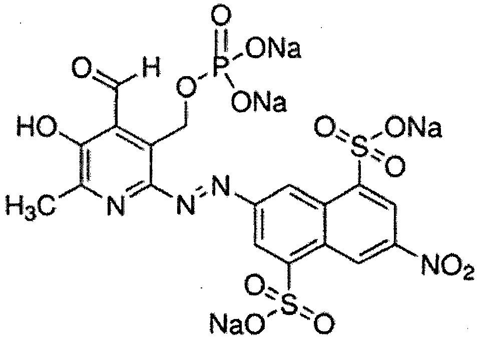

### 70. (11bR)-2,6-Di-9-phenanthrenyl-4-hydroxy-dinaphtho[2,1-d:1',2'-f][1,3,2]di-oxaphosphepin-4-oxid

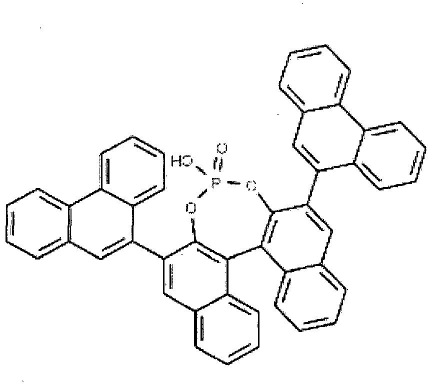

### 71. 8-Bromo-cyclic adenosin diphosphat ribose

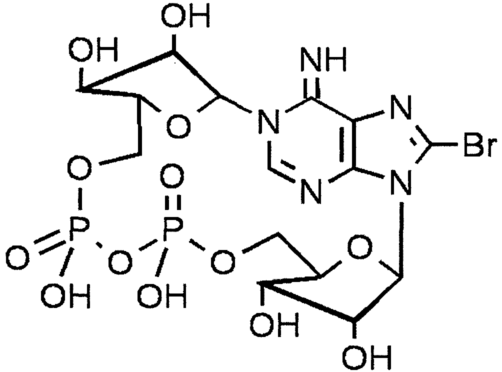

### 72. Phytic acid

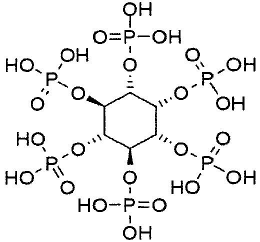

### 73. Trypan Blau

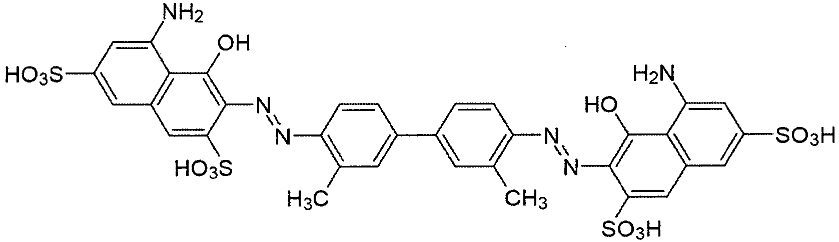

### 74. Glucose-6-phosphat bzw. andere Phosphorsäureester von Zuckern

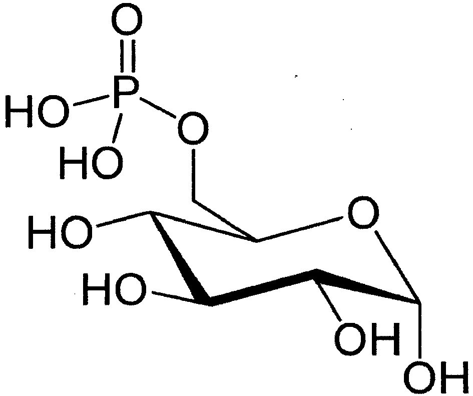

### 75. Natürlich vorkommenden und synthetische Nukleotide wie

- Adenosinmonophosphat (AMP) 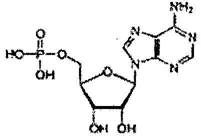
- Adenosindiphosphat (ADP) 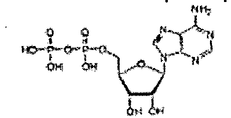
- Adenosintriphosphat (ATP) 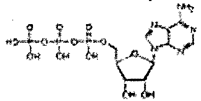
- Guanosinmonophosphat (GMP) 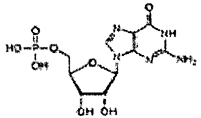
- Guanosindiphosphat (GDP) 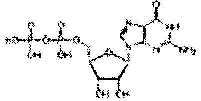
- Guanosintriphosphat (GTP) 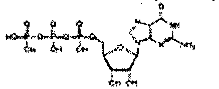
- Cytidinmonophosphat (CMP) 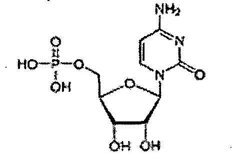
- Cytidindiphosphat (CDP) 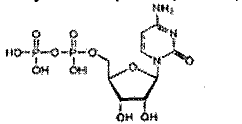
- Cytidintriphosphat (CTP) 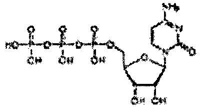
- Uridinmonophosphat (UMP) 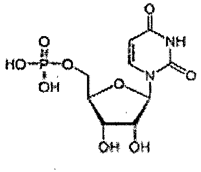
- Uridindiphosphat (UDP) 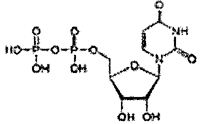 Uridintriphosphat (UTP) 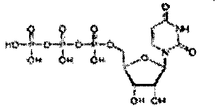
- Desoxyadenosinmonophosphat (dAMP) 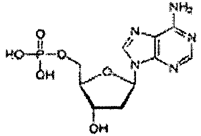
- Desoxyadenosindiphosphat (dADP) 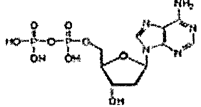
- Desoxyadenosintriphosphat (dATP) 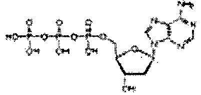
- Desoxyguanosinmonophosphat (dGMP) 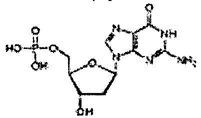
- Desoxyguanosindiphosphat (dGDP) 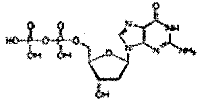
- Desoxyguanosintriphosphat (dGTP) 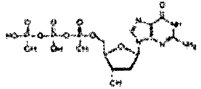
- Desoxycytidinmonophosphat (dCMP) 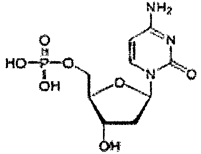
- Desoxycytidindiphosphat (dCDP) 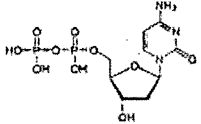
- Desoxycytidintriphosphat (dCTP) 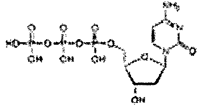
- Desoxythymidinmonophosphat (dTMP) 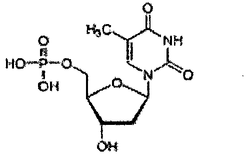
- Desoxythymidindiphosphat (dTDP) 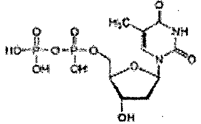
- Desoxythymidintriphosphat (dTTP)

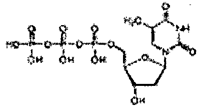

Ferner können auch leuchtstoffmarkierte Nukleotide zur Synthese von leuchtenden Hybridverbindungen eingesetzt werden. Solche sind im Handel erhältlich, beispielsweise von Life Technologies (unter der Bezeichnung ALEXA) oder Dyomics (unter der Bezeichnung DY). Die Farbstoffe sind IR-Fluoreszenzfarbstoffe. Insofern sichtbares Licht im Gewebe sehr schnell bei wenigen Mikrometern Gewebedicke absorbiert wird, ist eine IR-Emission in der Medizin infolge der tiefen Gewebe-Durchdringung von IR-Licht besonders vorteilhaft. Diese IR-Farbstoffe sind Standardfarbstoffe für die medizinische Anwendung. Die nachstehend beispielhaft aufgeführten leuchtstoffmarkierten Nukleotide enthalten alle eine Phosphatgruppe und lassen sich problemlos in die erfindungsgemäßen Hybridverbindungen einbauen. Produktnamen (z.B. ALEXA bzw. DY), die Anregungs- und Emissionswellenlänge und zum Teil die Summenformel der Verbindungen sind nachstehend angegeben.

| Leuchtstoff-modifiziertes Nukleotid | Molare Masse in g mol⁻¹ | Summenformel | Exc/Em in nm |
|---|---|---|---|
| DY-630-dUTP | 1163.78 | C₄₈H₆₀N₅O₁₉P₃S * 4Li | 636/657 (in Ethanol) |
| DY-631-dUTP | 1362.94 | C₅₄H₇₀N₆O₂₃P₃S₂ * 5Li | 637/658 (in Ethanol) |
| DY-632-dUTP | 1462.96 | C₅₅H₇₁N₆O₂₆P₃S₃Li₆ | 637/657 (in Ethanol) |
| DY-633-dUTP | 1263.80 | C₄₉H₆₁N₅O₂₂P₃S₂ * 5Li | 637/657 (in Ethanol) |
| DY-634-dUTP | 1562.98 | C₅₆H₇₂N₆O₂₉P₃S₄Li₇ | 635/658 (in Ethanol) |
| DY-635-dUTP | 1187.80 | C₅₀H₆₀N₅O₁₉P₃SLi₄ | 647/671 (in Ethanol) |
| DY-636-dUTP | 1386.96 | C₅₆H₇₀N₆O₂₃P₃S₂ * 5Li | 645/671 (in Ethanol) |
| DY-647P1-dUTP | 1221.72 | C₄₆H₅₅N₅O₂₂P₃S₂ * 5Li | 653/672 (in Ethanol) |
| DY-648P1-dUTP | 1335.77 | C₄₈H₅₈N₅O₂₅P₃S₃* 6Li | 653/672 (in Ethanol) |
| DY-650-dUTP | 1215.86 | C₅₂H₆₄N₅O₁₉P₃SLi₄ | 653/674 (in Ethanol) |
| DY-651-dUTP | 1415.01 | C₆₆H₇₄N₆O₂₃P₃S₂ * 5Li | 656/678 (in Ethanol) |
| DY-652-dUTP | 1515.03 | C₅₉H₇₅N₆O₂₆P₃S₃Li₆ | 654 /675 (in Ethanol) |
| DY-677-dUTP | 1535.02 | C₆₁H₇₁N₆O₂₆P₃S₃Li₂ | 673/694 (in Ethanol) |
| DY-678-dUTP | | | 674/694 (in Ethanol) |
| DY-679P1-dUTP | | | 679/697 (in PBS) |
| DY-681-dUTP | 1362.94 | C₅₄H₇₀N₆O₂₃P₃S₂Li₅ | 691/708 (in Ethanol) |
| DY-781-dUTP | 1388.98 | C₅₆H₇₂N₆O₂₃P₃S₂Li₅ | 783/800 (in Ethanol) |

| Leuchtstoff-modifiziertes Nukleotid | Exc/Em in nm |
|---|---|
| Alexa Fluor 546-14-dUTP | 555/570 |
| Alexa Fluor 555-aha-dUTP | 555/570 |
| Alexa Fluor 555-aha-dCTP | 555/570 |
| Alexa Fluor 546-16-OBEA-dCTP | 555/570 |
| Alexa Fluor 546-14-UTP | 555/570 |
| Alexa Fluor 568-5-dUTP | 575/600 |
| Texas Red-12-dUTP | 595/610 |
| Alexa Fluor 594-5-dUTP | 590/615 |
| Alexa Fluor 647-aha-dUTP | 650/670 |
| Alexa Fluor 647-aha-dCTP | 650/670 |
| Alexa Fluor 647-12-OBEA-dCTP | 650/670 |

## Patentansprüche

1. Anorganisch-organische Hybridverbindung als Ionenverbindung, aufgebaut aus einem anorganischen Metallkation, ausgewählt aus Mg²⁺, Ca²⁺, Sr²⁺, Ba²⁺, Zn²⁺, Zr⁴⁺, [ZrO]²⁺, [HfO]²⁺, Sc³⁺, y³⁺, Gd³⁺, La³⁺, Fe³⁺, Bi³⁺ oder einem Lanthanoid, und einem organischen Wirkstoffanion, welches mindestens eine Phosphat-, Phosphonat-, Sulfat-, Sulfonat, Carbonat- oder Carboxylatgruppe als funktionelle Gruppe enthält, wobei die Verbindung eine molare Löslichkeit von ≤ 10⁻² mol/l in Wasser aufweist und einen Partikeldurchmesser im Bereich von 1 bis 100 nm aufweist.

2. Anorganisch-organische Hybridverbindung gemäß Anspruch 1, welche weiter ein Fluoreszenzfarbstoffanion, das eine Phosphat-, Phosphonat-, Sulfat-, Sulfonat, Carbonat- oder Carboxylatgruppe als funktionelle Gruppe trägt, umfasst.

3. Anorganisch-organische Hybridverbindung nach Anspruch 1 oder 2, welche in röntgenamorpher Form vorliegt.

4. Anorganisch-organische Hybridverbindung nach einem der Ansprüche 1 bis 3, welche mit einem Lanthanoid, ausgewählt aus Ce, Pr, Nd, Sm, Eu, Gd, Tb, Dy, Ho, Er, Tm, Yb oder Lu, einem Übergangsmetall, ausgewählt aus Cr, Mn, Cu, Zn, Y, Ag oder Cd, einem Hauptgruppenelement, ausgewählt aus Sn, Sb, Pb oder Bi, oder einem komplexen Anion, ausgewählt aus [VO₄]³⁻, [MoO4]³⁻ oder [WO₄]³⁻, dotiert ist.

5. Anorganisch-organische Hybridverbindung nach einem der Ansprüche 1 bis 4, dessen Wirkstoffanion gegen chronische Entzündungen, Asthma, Rheuma, Arthritis, Multiple Sklerose, Entzündungen, Tumorerkrankungen, Malaria, Tuberkulose, Angina Pectoris oder koronare Ablagerungen gerichtet ist.

6. Anorganisch-organische Hybridverbindung nach einem der Ansprüche 1 bis 5, wobei das Wirkstoffanion von Acetaminophenphosphat, Betamethasonphosphat, Dexamethasonphosphat, Uridinmonophosphat, 5'-Fluor-2'-deoxyuridin-5'-monophosphat, Methylprednisolonphosphat, Triamcinolonphosphat, Estronphosphat, Testosteronphosphat, Estramustinphosphat, Codeinphosphat, Clindamycinphosphat, Thiaminpyrophosphat, Tiaminphosphat; Aracytidinmonophosphat, Cyclic-3',5'adenosinmonophosphat, Vidaribinphosphat, 9-[9-(Phosphonomethoxy)ethoxy]adenin, Fospropofol, Fosphenytoin, Phosphoryloxymethyloxymethylphenytoin, Phosphoryloxymethylphenylbutazon, Phosphoryloxymethyloxymethylphenylbutazon, Phosphoryloxymethylphenindion, Phosphoryloxymethyloxymethylphenindion, N-Phosphonooxymethylcinnarizin, N-Phosphonooxymethylloxapin, N-Phosphonooxymethylamiodaron, Alendronat, Canrenoat, Doxycyclin Hydrat, Doxorubicin Hydrochlorid, Aztreonam, Tigemonam, D-Glukosamin-6-sulfat, Colistin methansulfat, Cefsulodin, Fosamprenavir, Tenofovir, Adefovir, Combretastatin A-4 phosphat, Folsäure, Fosphenytoin, 2-Mercaptoethansulfonat / Mesna, Fosfomycin, Glyphosat, Glufosinat, Zolendronat, Aminotrimethylenphosphonsäure, Diethylentriaminpenta(methylenphosphonsäure), Ethylendiamintetra(methylenphosphonsäure), Fosbretabulin, α-Tocopherol phosphate, VAPOL hydrogenphosphate, Pyridoxal-5'-phosphate-6-(2'-naphthylazo-6'-nitro-4',8'-disulfonate), (11bR)-2,6-Di-9-phenanthrenyl-4-hydroxy-dinaphtho[2,1-d:1',2'-f] [1,3,2]dioxaphosphepin-4-oxide, 8-Bromo-cyclic adenosine diphosphate ribose, Phytinsäure, Glucose-6-phosphat bzw. andere Phosphorsäureester von Zuckern oder natürlich vorkommende und synthetische Nukleotide einschließlich Adenosinmonophosphat (AMP), Adenosindiphosphat (ADP), Adenosintriphosphat (ATP), Guanosinmonophosphat (GMP), Guanosindiphosphat (GDP), Guanosintriphosphat (GTP), Cytidinmonophosphat (CMP), Cytidindiphosphat (CDP), Cytidintriphosphat (CTP), Uridinmonophosphat (UMP), Uridindiphosphat (UDP), Uridintriphosphat (UTP), Desoxyadenosinmonophosphat (dAMP), Desoxyadenosindiphosphat (dADP), Desoxyadenosintriphosphat (dATP), Desoxyguanosinmonophosphat (dGMP), Desoxyguanosindiphosphat (dGDP), Desoxyguanosintriphosphat (dGTP), Desoxycytidinmonophosphat (dCMP), Desoxycytidindiphosphat (dCDP), Desoxycytidintriphosphat (dCTP), Desoxythymidinmonophosphat (dTMP), Desoxythymidindiphosphat (dTDP) oder Desoxythymidintriphosphat (dTTP)) abgeleitet ist.

7. Anorganisch-organische Hybridverbindung nach einem der Ansprüche 1 bis 6, welche weiter mit einem Antikörper, Peptid oder Oligonukleotid funktionalisiert ist.

8. Anorganisch-organische Hybridverbindung nach einem der Ansprüche 1 bis 7, wobei das organische Fluoreszenzfarbstoffanion von Fluoreszenzfarbstoffen abgeleitet ist, ausgewählt aus der Gruppe, bestehend aus 1,1'-Diethyl-2,2'-cyaniniodid, 1,2-Diphenylacetylen, 1,4-Diphenylbutadien, 1,4-Diphenylbutadien, 1,6-Diphenylhexatrien, 2,5-Diphenyloxazol, 2-Methylbenzoxazol, 4',6-Diamidino-2-phenylindol (DAPI), 4-(Dicyanomethylen)-2-methyl-6-(p-dimethylaminostyryl)-4H-pyran (DCM), 4-Dimethylamino-4'-nitrostilben, 5,10,15-Triphenylcorrol, 5,10,15-Tris(pentafluorophenyl)corrol, 5,10-Diarylchlorin, 5,10-Diarylkupferchlorin, 5,10-Diarylkupferoxochlorin, 5,10-Diarylmagnesiumoxochlorin, 5,10-Diaryloxochlorin, 5,10-Diarylzinkchlorin, 5,10-Diarylzinkooxochlorin, 7-Benzylamino-4-nitrobenz-2-oxa-1,3-diazol, 7-Methoxycumarin-4-essigsäure, 9,10-Bis(phenylethinyl)anthracen, 9,10-Diphenylanthracen, Acridinorange, Acridingelb, Adenin, Anthracen, Anthrachinon, Auramin O, Azobenzol, Bakteriochlorophyll A, Benzochinon, Betacarotin, Bilirubin, Biliverdindimethylester, Biphenyl, Bis(5-mesityldipyrrinato)zink, Bis(5-phenyldipyrrinato)zink, Borsubphtalocyaninchlorid, Chlorin E6, Chlorophyll A, Chlorophyll B, cis-Stilben, Cumarin sowie dessen Derivaten, Cresylviolettperchlorat, Cryptocyanin, Kristallviolett, Cytosin, Dansylglycin, Diprotonated-tetraphenylporphyrin, Eosin sowie dessen Derivaten, Ethyl-(p-dimethylamino)-benzoat, Ferrocen, Fluorescein sowie dessen Derivaten, beispielsweise Methylfluorescein, Resorufin, Amaranth, Aluminium(III)-Phthalocyanin-Chlorid Tetrasulfonsäure, Trypan Blau, Guanin, Hematin, Histidin, Hoechst 33258, Indocarbocyanine sowie dessen Derivaten, Lucifergelb CH, Magnesiumoctaethylporphyrin, Magnesiumphthalocyanin, Magnesiumtetramesitylporphyrin, Magnesiumtetraphenylporphyrin, Malachitgrün, Merocyanin, N,N'-Difluoroboryl-1,9-dimethyl-5-(4-iodophenyl)-dipyrrin, N,N'-Difluoroboryl-1,9-dimethyl-5-[(4-(2-trimethylsilylethinyl), N,N'-Difluoroboryl-1,9-dimethyl-5-phenydipyrrin, Tetraphenylporphyrin, Naphthalin, Nilblau, Nilrot, Octaethylporphyrin, Oxacarbocyanin sowie dessen Derivaten, Oxazin sowie dessen Derivaten, p-Quarterphenyl, p-Terphenyl, Perylen sowie dessen Derivaten, Phenol, Phenylalanin, Phenyldipyrrin, Pheophorbid, Phthalocyanin, Pinacyanoliodid, Piroxicam, Porphin, Proflavin, Protoporphyrin-IX-dimethylester, Pyren, Pyropheophorbid sowie dessen Derivaten, Pyrrol, Chinin, Rhodamin sowie dessen Derivaten, Riboflavin, Bengalrot, Squarylium dye III, TBP-beta-octa(COOBu)-Fb, TBP-beta-octa(COOBu)-Pd, TBP-beta-octa(COOBu)-Zn, TBP-meso-tetraphenyl-beta-octa(COOMe)-Fb, TBP-meso-tetraphenyl-beta-octa(COOMe)-Pd, TBP-meso-tetraphenyl-beta-octa(COOMe)-Zn, TCPH-meso-tetra(4-COOMe-phenyl)-Fb, TCPH-meso-tetra(4-COOMe-phenyl)-Pd, TCPH-meso-tetra(4-COOMe-phenyl)-Zn, Tetra-t-butylazaporphin, Tetra-t-butylnaphthalocyanin, Tetrakis(2,6-dichlorophenyl)porphyrin, Tetrakis(o-aminophenyl)porphyrin, Tetramesitylporphyrin, Tetraphenylporphyrin, Tetraphenylsapphyrin, Thiacarbocyanin sowie dessen Derivaten, Thymin, trans-Stilben, Tris(2,2'-bipyridyl)ruthenium(II), Tryptophan, Thyrosin, Uracil, Vitamin B12, Zinkoctaethylporphyrin, Phthalocyanin sowie dessen Derivaten, Porphyrin sowie dessen Derivaten einschließlich Tetra(o-amidophosphonatophenyl)porphyrin, und Umbelliferon,
wobei die organischen Fluoreszenzfarbstoffe, die als solche keine Phosphat-, Phosphonat-, Sulfat-, Sulfonat, Carbonat- oder Carboxylatgruppe aufweisen, mit mindestens einer von diesen funktionellen Gruppen modifiziert worden sind.

9. Verfahren zur Herstellung einer anorganisch-organischen Hybridverbindung nach einem der Ansprüche 1 bis 8, umfassend die Schritte
(a) das Bereitstellen einer Lösung einer organischen Wirkstoffverbindung, die eine oder mehrere funktionelle Gruppen, ausgewählt aus Phosphat-, Phosphonat-, Sulfat-, Sulfonat, Carbonat- oder Carboxylatgruppen, aufweist, wobei die Lösung gegebenenfalls weiter mindestens ein Anion, ausgewählt aus Phosphat-, Phosphonat-, Sulfat-, Sulfonat, Carbonat- oder Carboxylat, enthält,
(b) das Bereitstellen einer Lösung eines löslichen Metallsalzes, enthaltend Metallkationen, ausgewählt aus Mg²⁺, Ca²⁺, Sr²⁺, Ba²⁺, Zn²⁺, Zr⁴⁺, [ZrO]²⁺, [HfO]²⁺, Sc³⁺, y³⁺, Gd³⁺, La³⁺, Fe³⁺, Bi³⁺ oder einem Lanthanoid,
(c) das Vereinigen der beiden Lösungen unter Rühren, um die Hybridverbindung auszufällen, und
(d) das Isolieren und/oder Aufreinigen der ausgefällten Hybridverbindung.

10. Verfahren gemäß Anspruch 9, wobei zu der in Schritt (a) bereitgestellten Lösung weiter ein organischer Fluoreszenzfarbstoff, welcher eine oder mehrere funktionelle Gruppen, ausgewählt aus Phosphat-, Phosphonat-, Sulfat-, Sulfonat, Carbonat- oder Carboxylatgruppen, aufweist, zugegeben wird.

## Claims

1. An inorganic-organic hybrid compound as ionic compound, composed of an inorganic metal cation selected from Mg²⁺, Ca²⁺, Sr²⁺, Ba²⁺, Zn²⁺, Zr⁴⁺, [ZrO]²⁺, [HfO]²⁺, Sc³⁺, Y³⁺, Gd³⁺, La³⁺, Fe³⁺, Bi³⁺ or a lanthanoid, and of an organic active ingredient anion which comprises at least one phosphate, phosphonate, sulfate, sulfonate, carbonate or carboxylate group as functional group, the compound having a molar solubility of ≤ 10⁻² mol/l in water and having a particle diameter in the range from 1 to 100 nm.

2. The inorganic-organic hybrid compound as claimed in claim 1, further comprising a fluorescent dye anion which carries a phosphate, phosphonate, sulfate, sulfonate, carbonate or carboxylate group as functional group.

3. The inorganic-organic hybrid compound as claimed in claim 1 or 2, which is in X-ray-amorphous form.

4. The inorganic-organic hybrid compound as claimed in any of claims 1 to 3, which is doped with a lanthanoid selected from Ce, Pr, Nd, Sm, Eu, Gd, Tb, Dy, Ho, Er, Tm, Yb or Lu, with a transition metal selected from Cr, Mn, Cu, Zn, Y, Ag or Cd, with a main group element selected from Sn, Sb, Pb or Bi, or with a complex anion selected from [VO₄]³⁻, [MoO₄]³⁻ or [WO₄]³⁻.

5. The inorganic-organic hybrid compound as claimed in any of claims 1 to 4, whose active ingredient anion is directed against chronic inflammation, asthma, rheumatism, arthritis, multiple sclerosis, inflammation, tumor disorders, malaria, tuberculosis, angina pectoris or coronary deposits.

6. The inorganic-organic hybrid compound as claimed in any of claims 1 to 5, the active ingredient anion being derived from acetaminophen phosphate, betamethasone phosphate, dexamethasone phosphate, uridine monophosphate, 5'-fluoro-2'-deoxyuridine 5'-monophosphate, methylprednisolone phosphate, triamcinolone phosphate, estrone phosphate, testosterone phosphate, estramustine phosphate, codeine phosphate, clindamycin phosphate, thiamine pyrophosphate, thiamine phosphate; aracytidine monophosphate, cyclic 3',5'-adenosine monophosphate, vidaribine phosphate, 9-[9-(phosphonomethoxy)ethoxy]adenine, fospropofol, fosphenytoin, phosphoryloxymethyloxymethylphenytoin, phosphoryloxymethylphenylbutazone, phosphoryloxymethyloxymethylphenylbutazone, phosphoryloxymethylphenindione, phosphoryloxymethyloxymethylphenindione, N-phosphonooxymethylcinnarizine, N-phosphonooxymethylloxapine, N-phosphonooxymethylamiodarone, alendronate, canrenoate, doxycyclin hydrate, doxorubicin hydrochloride, aztreonam, tigemonam, D-glucosamine 6-sulfate, colistin methane sulfate, cefsulodin, fosamprenavir, tenofovir, adefovir, combretastatin A-4 phosphate, folic acid, 2-mercaptoethansulfonate / mesna, fosfomycin, glyphosate, glufosinate, zolendronate, aminotrimethylenephosphonic acid, diethylenetriamine-penta(methylenephosphonic acid), ethylenediaminetetra(methylenephosphonic acid), fosbretabulin, α-tocopherol phosphate, VAPOL hydrogenphosphate, pyridoxal 5'-phosphate 6-2'-naphthylazo-6'-nitro-4',8'-disulfonate), (11bR)-2,6-di-9-phenanthrenyl-4-hydroxydinaphtho[2,1-d:1',2'-f] [1,3,2]dioxaphosphepine 4-oxide, 8-bromo cyclic adenosine diphosphate ribose, phytic acid, glucose 6-phosphate and other phosphoric esters of sugars, or naturally occurring and synthetic nucleotides including adenosine monophosphate (AMP), adenosine diphosphate (ADP), adenosine triphosphate (ATP), guanosine monophosphate (GMP), guanosine diphosphate (GDP), guanosine triphosphate (GTP), cytidine monophosphate (CMP), cytidine diphosphate (CDP), cytidine triphosphate (CTP), uridine monophosphate (UMP), uridine diphosphate (UDP), uridine triphosphate (UTP), deoxyadenosine monophosphate (dAMP), deoxyadenosine diphosphate (dADP), deoxyadenosine triphosphate (dATP), deoxyguanosine monophosphate (dGMP), deoxyguanosine diphosphate (dGDP), deoxyguanosine triphosphate (dGTP), deoxycytidine monophosphate (dCMP), deoxycytidine diphosphate (dCDP), deoxycytidine triphosphate (dCTP), deoxythymidine monophosphate (dTMP), deoxythymidine diphosphate (dTDP) or deoxythymidine triphosphate (dTTP).

7. The inorganic-organic hybrid compound as claimed in any of claims 1 to 6, which is further functionalized with an antibody, peptide or oligonucleotide.

8. The inorganic-organic hybrid compound as claimed in any of claims 1 to 7, the organic fluorescent dye anion being derived from fluorescent dyes selected from the group consisting of 1,1'-diethyl-2,2'-cyanine iodide, 1,2-diphenylacetylene, 1,4-diphenylbutadiene, 1,6-diphenylhexatriene, 2,5-diphenyloxazole, 2-methylbenzoxazole, 4',6-diamidino-2-phenylindole (DAPI), 4-(dicyanomethylene)-2-methyl-6-(p-dimethylaminostyryl)-4H-pyran (DCM), 4-dimethylamino-4'-nitrostilbene, 5,10,15-triphenylcorrole, 5,10,15-tris(pentafluorophenyl)corrole, 5,10-diarylchlorin, 5,10-diarylcopper chlorin, 5,10-diarylcopper oxochlorin, 5,10-diarylmagnesium oxochlorin, 5,10-diaryloxochlorin, 5,10-diarylzinc chlorin, 5,10-diarylzinc oxochlorin, 7-benzylamino-4-nitrobenz-2-oxa-1,3-diazole, 7-methoxycoumarin-4-acetic acid, 9,10-bis(phenylethynyl)anthracene, 9,10-diphenylanthracene, acridine orange, acridine yellow, adenine, anthracene, anthraquinone, auramine O, azobenzene, bacteriochlorophyll A, benzoquinone, beta-carotene, bilirubin, biliverdin dimethyl ester, biphenyl, bis(5-mesityldipyrrinato)zinc, bis(5-phenyldipyrrinato)zinc, boron subphthalocyanine chloride, chlorin E6, chlorophyll A, chlorophyll B, cis-stilbene, coumarin and its derivatives, cresyl violet perchlorate, cryptocyanine, crystal violet, cytosine, dansylglycine, diprotonated tetraphenylporphyrin, eosine and its derivatives, ethyl (p-dimethylamino)benzoate, ferrocene, fluorescein and its derivatives, as for example methylfluorescein, resorufin, amaranth, aluminum(III)-phthalocyanine chloride tetrasulfonic acid, trypan blue, guanine, hematin, histidine, Hoechst 33258, indocarbocyanine and its derivatives, lucifer yellow CH, magnesium octaethylporphyrin, magnesium phthalocyanine, magnesium tetramesitylporphyrin, magnesium tetraphenylporphyrin, malachite green, merocyanine, N,N'-difluoroboryl-1,9-dimethyl-5-(4-iodophenyl)dipyrrin, N,N'-difluoroboryl-1,9-dimethyl-5-[(4-(2-trimethylsilylethynyl), N,N'-difluoroboryl-1,9-dimethyl-5-phenyldipyrrin, tetraphenylporphyrin, naphthalene, nile blue, nile red, octaethylporphyrin, oxacarbocyanine and its derivatives, oxazine and its derivatives, p-quaterphenyl, p-terphenyl, perylene and its derivatives, phenol, phenylalanine, phenyldipyrrin, pheophorbide, phthalocyanine, pinacyanol iodide, piroxicam, porphin, proflavin, protoporphyrin IX dimethyl ester, pyrene, pyropheophorbide and its derivatives, pyrrol, quinine, rhodamine and its derivatives, riboflavin, bengal red, squarylium dye III, TBP beta-octa(COOBu)-Fb, TBP beta-octa(COOBu)-Pd, TBP beta-octa(COOBu)-Zn, TBP meso-tetraphenyl-beta-octa(COOMe)-Fb, TBP meso-tetraphenyl-beta-octa(COOMe)-Pd, TBP meso-tetraphenyl-beta-octa(COOMe)-Zn, TCPH meso-tetra(4-COOMe-phenyl)-Fb, TCPH meso-tetra(4-COOMe-phenyl)-Pd, TCPH meso-tetra(4-COOMe-phenyl)-Zn, tetra-tert-butylazaporphin, tetra-tert-butylnaphthalocyanine, tetrakis(2,6-dichlorophenyl)porphyrin, tetrakis(o-aminophenyl)porphyrin, tetramesitylporphyrin, tetraphenylporphyrin, tetraphenylsapphyrin, thiacarbocyanine and its derivatives, thymine, trans-stilbene, tris(2,2'-bipyridyl)ruthenium(II), tryptophan, thyrosine, uracil, vitamin B12, zinc octaethylporphyrin, phthalocyanine and its derivatives, porphyrin and its derivatives, including tetra(o-amidophosphonatophenyl)porphyrin, and umbelliferone,
where the organic fluorescent dyes which do not as such have a phosphate, phosphonate, sulfate, sulfonate, carbonate or carboxylate group have been modified with at least one of these functional groups.

9. A process for preparing an inorganic-organic hybrid compound as claimed in any of claims 1 to 8, comprising the steps of
(a) providing a solution of an organic active ingredient compound which has one or more functional groups selected from phosphate, phosphonate, sulfate, sulfonate, carbonate or carboxylate groups, the solution optionally further comprising at least one anion selected from phosphate, phosphonate, sulfate, sulfonate, carbonate or carboxylate,
(b) providing a solution of a soluble metal salt comprising metal cations selected from Mg²⁺, Ca²⁺, Sr²⁺, Ba²⁺, Zn²⁺, Zr⁴⁺, [ZrO]²⁺, [HfO]²⁺, Sc³⁺, Y³⁺, Gd³⁺, La³⁺, Fe³⁺, Bi³⁺ or a lanthanoid,
(c) combining the two solutions with stirring, to precipitate the hybrid compound, and
(d) isolating and/or purifying the precipitated hybrid compound.

10. The process as claimed in claim 9, where the solution provided in step (a) is further admixed with an organic fluorescent dye which has one or more functional groups selected from phosphate, phosphonate, sulfate, sulfonate, carbonate or carboxylate groups.

## Revendications

1. Composé hybride inorganique-organique en tant que composé ionique, composé d'un cation métallique inorganique choisi parmi Mg²⁺, Ca²⁺, Sr²⁺, Ba²⁺, Zn²⁺, Zr⁴⁺, [ZrO]²⁺, [HfO]²⁺, Sc³⁺, Y³⁺, Gd³⁺, La³⁺, Fe³⁺, Bi³⁺ ou un lanthanoïde, et un anion organique de substance active, qui comporte au moins un groupe phosphate, phosphonate, sulfate, sulfonate, carbonate ou carboxylate comme groupe fonctionnel, où le composé présente une solubilité molaire ≤ 10⁻² mole/l dans l'eau et un diamètre particulaire situé dans l'intervalle allant de 1 à 100 nm.

2. Composé hybride inorganique-organique selon la revendication 1, qui comprend en outre, un anion de colorant fluorescent, qui porte un groupe phosphate, phosphonate, sulfate, sulfonate, carbonate ou carboxylate comme groupe fonctionnel

3. Composé hybride inorganique-organique selon la revendication 1 ou 2, sous une forme amorphe aux rayons X.

4. Composé hybride inorganique-organique selon l'une quelconque des revendications 1 à 3, qui est dopé par un lanthanoïde choisi parmi Ce, Pr, Nd, Sm, Eu, Gd, Tb, Dy, Ho, Er, Tm, Yb ou Lu, avec un métal de transition choisi parmi Cr, Mn, Cu, Zn, Y, Ag ou Cd, avec un élément de groupe principal choisi parmi Sn, Sb, Pb ou Bi, ou un anion complexe choisi parmi [VO₄]³⁻, [MoO₄]³⁻ or [WO₄]³⁻.

5. Composé hybride inorganique-organique selon l'une quelconque des revendications 1 à 4, dont l'anion de substance active cible des inflammations chroniques, l'asthme, le rhumatisme, l'arthrite, la sclérose en plaques, des inflammations, des tumeurs, la malaria, la tuberculose, l'angine de poitrine ou des dépôts coronaires.

6. Composé hybride inorganique-organique selon l'une quelconque des revendications 1 à 5, où l'anion de substance active est dérivé du phosphate d'acétaminophène, du phosphate de bêtaméthasone, du phosphate de dexaméthasone, du monophosphate d'uridine, du 5'-monophosphate de 5'-fluoro-2'-désoxyuridine, du phosphate de méthylprednisolone, du phosphate de triamcinolone, du phosphate d'estrone, du phosphate de testostérone, du phosphate d'estramustine, du phosphate de codéine, du phosphate de clindamycine, du pyrophosphate de thiamine, du phosphate de thiamine; du monophosphate d'aracytidine, du monophosphate de 3',5'-adénosine cyclique, du phosphate de vidaribine, du 9-[9-(phosphonométhoxy)éthoxy]adénine, du fospropofol, de la fosphénytoïne, de la phosphoryloxyméthyloxyméthylphénytoïne, de la phosphoryloxyméthylphénylbutazone, de la phosphoryloxyméthyloxyméthyl-phénylbutazone, de la phosphoryloxyméthylphénindione, de la phosphoryloxy-méthyloxyméthylphénindione, de la N-phosphonooxyméthylcinnarizine, de la N-phosphonooxyméthylloxapine, de la N-phosphonooxyméthylamiodarone, de l'alendronate, du canrénoate, de l'hydrate de doxycycline, du chlorhydrate de doxorubicine, de l'aztréonam, du tigémonam, du 6-sulfate de D-glucosamine, du méthanesulfate de colistine, de la cefsulodine, du fosamprénavir, du ténofovir, de l'adéfovir, du phosphate de la combretastatine A4, de l'acide folique, du 2-mercapto-éthansulfonate / mesna, de la fosfomycine, du glyphosate, du glufosinate, du zolendronate, de l'acide aminotriméthylenephosphonique, de l'acide diéthylène-triaminepenta(méthylènephosphonique), de l'acide éthylènediaminetétra(méthylène-phosphonique), de la fosbrétabuline, du phosphate d'a-tocophérol, de l'hydrogénophosphate de VAPOL, du pyridoxal 5'-phosphate 6-2'-naphtylazo-6'-nitro-4',8'-disulfonate), du 4-oxyde de (11bR)-2,6-di-9-phénanthrényl-4-hydroxydinaphtho[2,1-d:1',2'-f] [1,3,2]dioxaphosphépine, du 8-bromoadénosine cyclique diphosphate ribose, de l'acide phytique, du glucose 6-phosphate et d'autres esters phosphoriques de sucres, ou des nucléotides naturels et synthétiques incluant l'adénosine monophosphate (AMP), l'adénosine diphosphate (ADP), l'adénosine triphosphate (ATP),la guanosine monophosphate (GMP), la guanosine diphosphate (GDP), la guanosine triphosphate (GTP), la cytidine monophosphate (CMP), la cytidine diphosphate (CDP), la cytidine triphosphate (CTP), l'uridine monophosphate (UMP), l'uridine diphosphate (UDP), l'uridine triphosphate (UTP), la désoxyadénosine monophosphate (dAMP), la désoxyadsnosine diphosphate (dADP), la désoxyadénosine triphosphate (dATP), la désoxyguanosine monophosphate (dGMP), la désoxyguanosine diphosphate (dGDP), la désoxyguanosine triphosphate (dGTP), la désoxycytidine monophosphate (dCMP), la désoxycytidine diphosphate (dCDP), la désoxycytidine triphosphate (dCTP), la désoxythymidine monophosphate (dTMP), la désoxythymidine diphosphate (dTDP) ou la désoxythymidine triphosphate (dTTP).

7. Composé hybride inorganique-organique selon l'une quelconque des revendications 1 à 6, qui est en outre fonctionnalisé par un anticorps, un peptide ou un oligonucléotide.

8. Composé hybride inorganique-organique selon l'une quelconque des revendications 1 à 7, où l'anion de colorant fluorescent est dérivé de colorants fluorescents choisis parmi le groupe consistant en l'iodure de 1,1'-diéthyl-2,2'-cyanine, le 1,2-diphénylacétylène, le 1,4-diphénylbutadiène, le 1,6-diphénylhexatriène, le 2,5-diphényloxazole, le 2-méthylbenzoxazole, le 4',6-diamidino-2-phénylindole (DAPI), le 4-(dicyanométhylène)-2-méthyl-6-(p-diméthylaminostyryl)-4H-pyranne (DCM), le 4-diméthylamino-4'-nitrostilbène, le 5,10,15-triphénylcorrole, le 5,10,15-tris(pentafluoro-phényl)corrole, la 5,10-diarylchlorine, la 5,10-diarylcuivre-chlorine, la 5,10-diarylcuivre oxochlorine, la 5,10-diarylmagnésium oxochlorine, la 5,10-diaryloxochlorine, la 5,10-diarylzinc chlorine, la 5,10-diarylzinc oxochlorine, le 7-benzylamino-4-nitrobenz-2-oxa-1,3-diazole, l'acide 7-méthoxycoumarine-4-acétique, le 9,10-bis(phényléthynyl)-anthracène, le 9,10-diphénylanthracène, l'acridine orange, l'acridine jaune, l'adénine, l'anthracène, l'anthraquinone, l'auramine O, l'azobenzène, la bactériochlorophylle A, la benzoquinone, le bêta-carotène, la bilirubine, l'ester diméthylique de la biliverdine, le biphényle, le bis(5-mésityldipyrrinato)zinc, le bis(5-phényldipyrrinato)zinc, le chlorure de bore subphthalocyanine, la chlorine E6, la chlorophylle A, la chlorophylle B, le cis-stilbène, la coumarine et ses dérivés, le perchlorate de crésyle violet, la cryptocyanine, le cristal violet, la cytosine, la dansylglycine, la tétraphenylporphyrine diprotonée, l'éosine et ses dérivés, le (p-diméthylamino)benzoate d'éthyle, le ferrocène, la fluorescéine et ses dérivés, par exemple la méthylfluorescéine, la résorufine, l'amaranthe, le chlorure d'acide tétrasulfonique de phthalocyanine d'aluminium(III), le bleu de trypan, la guanine, l'hématine, l'histidine, le Hoechst 33258, l'indocarbocyanine et ses dérivés, le jaune lucifer CH, l'octaéthylporphyrine de magnésium, la phthalocyanine de magnésium, la tétramésitylporphyrine de magnésium, la tétraphénylporphyrine de magnésium, le vert malachite, la mérocyanine, la N,N'-difluoroboryl-1,9-diméthyl-5-(4-iodophényl)dipyrrine, la N,N'-difluoroboryl-1,9-diméthyl-5-[(4-(2-triméthylsilyléthynyl), N,N'-difluoroboryl-1,9-diméthyl-5-phényldipyrrine, la tétraphénylporphyrine, le naphtalène, le bleu de nil, le rouge de nil, l'octaéthylporphyrine, l'oxacarbocyanine et ses dérivés, l'oxazine et ses dérivés, le p-quaterphényle, le p-terphényle, le perylène et ses dérivés, le phénol, la phénylalanine, la phényldipyrrine, le phéophorbide, la phthalocyanine, l'iodure de pinacyanol, le piroxicam, la porphine, la proflavine, l'ester diméthylique de protoporphyrine IX, le pyrène, le pyrophéophorbide et ses dérivés, le pyrrole, la quinine, la rhodamine est ses dérivés, la riboflavine, le rouge Bengale, le colorant squarylium III, le TBP bêta-octa(COOBu)-Fb, le TBP bêta-octa(COOBu)-Pd, le TBP bêta-octa(COOBu)-Zn, le TBP méso-tétraphényl-bêta-octa(COOMe)-Fb, le TBP méso-tétraphényl-bêta-octa(COOMe)-Pd, le TBP méso-tétraphényl-bêta-octa(COOMe)-Zn, le TCPH méso-tétra(4-COOMe-phényl)-Fb, le TCPH méso-tétra(4-COOMe-phényl)-Pd, le TCPH méso-tétra(4-COOMe-phényl)-Zn, la tétra-tert-butylazaporphine, la tétra-tert-butylnaphtalocyanine, la tétrakis(2,6-dichlorophényl)porphyrine, la tétrakis(o-aminophényl)porphyrine, la tétramésitylporphyrine, la tétraphénylporphyrine, la tétraphénylsapphyrine, la thiacarbocyanine et ses dérivés, la thymine, le trans-stilbène, le tris(2,2'-bipyridyl)ruthenium(II), le tryptophane, la thyrosine, l'uracile, la vitamine B12, l'octaéthylporphyrine de zinc, la phthalocyanine et ses dérivés, la porphyrine et ses dérivés, incluant le tétra(o-amidophosphonatophényl)porphyrine, et l'umbelliférone,
où les colorants fluorescents organiques, qui comme tels, ne comportent pas de groupe phosphate, phosphonate, sulfate, sulfonate, carbonate ou carboxylate, ont été modifiés par au moins l'un de ces groupes.

9. Procédé de préparation d'un composé hybride inorganique-organique selon l'une quelconque des revendications 1 à 8, comprenant les étapes de
(a) provision d'une solution d'un composé actif organique, qui présente un ou plusieurs groupes fonctionnels choisis parmi les groupes phosphate, phosphonate, sulfate, sulfonate, carbonate ou carboxylate, où la solution contient le cas échéant, au moins un anion choisi parmi un phosphate, un phosphonate, un sulfate, un sulfonate, un carbonate ou un carboxylate,
(b) provision d'une solution d'un sel métallique soluble contenant des cations métalliques choisis parmi Mg²⁺, Ca²⁺, Sr²⁺, Ba²⁺, Zn²⁺, Zr⁴⁺, [ZrO]²⁺, [HfO]²⁺, Sc³⁺, Y³⁺, Gd³⁺, La³⁺, Fe³⁺, Bi³⁺ ou un lanthanoïde,
(c) réunion des deux solutions sous agitation pour précipiter le composé hybride, et
(d) isolement et/ou purification du composé hybride précipité.

10. Procédé selon la revendication 9, où on ajoute en outre à la solution fournie à l'étape (a) un colorant fluorescent organique qui présente un ou plusieurs groupes fonctionnels choisis parmi les groupes phosphate, phosphonate, sulfate, sulfonate, carbonate ou carboxylate.
